# FASCICULE DE BREVET EUROPEEN

(11) **EP 0 743 988 B1**
(45) Date de publication et mention de la délivrance du brevet: **03.09.2003**
(21) Numéro de dépôt: 95909818.7
(22) Date de dépôt: 10.02.1995
(51) Int. Cl.: C12Q 1/68

(54) **PROCEDE D'ALIGNEMENT DE MACROMOLECULES PAR PASSAGE D'UN MENISQUE ET APPLICATIONS**
Verfahen zum Ausrichten von Makromolekülen durch Vorbeiführung eines Meniskus und Anwendung dafür
METHOD FOR ORDERING MACROMOLECULES BY MEANS OF A MOVING MENISCUS, AND USES THEREOF

(30) Priorité: 11.02.1994 FR 9401574; 17.06.1994 FR 9407444
(43) Date de publication de la demande: 27.11.1996
(60) Demande divisionnaire: 03011820.2
(73) Titulaire: INSTITUT PASTEUR, 75015 Paris (FR); CENTRE NATIONAL DE LA RECHERCHE SCIENTIFIQUE, 75016 Paris Cédex (FR)
(72) Inventeur: BENSIMON, David, F-75013 Paris (FR); BENSIMON, Aaron, F-92160 Antony (FR); HESLOT, François, F-78220 Viroflay (FR)
(74) Mandataire: Ahner, Francis
(86) Numéro de dépôt international: FR9500165
(87) Numéro de publication internationale: WO95021939

(56) Documents cités:
- EP-A- 0 127 438
- EP-A- 0 391 674
- EP-A- 0 578 148
- WO-A-93/22463
- DE-A- 3 717 210
- SCIENCE, vol. 262,no. 5130, 1 Octobre 1993 LANCASTER, PA US, pages 110-114, D.C.SCHWARTZ ET AL. cité dans la demande
- NATURE GENETICS, vol. 5, Septembre 1993 NEW YORK US, pages 17-21, I PARRA ET AL. cité dans la demande
- NUCLEIC ACIDS RESEARCH., vol. 22,no. 3, 11 Février 1994 ARLINGTON, VIRGINIA US, pages 492-497, R.M.ZIMMERMANN ET AL. cité dans la demande

## Description

La présente invention concerne une méthode d'alignement de macromolécules telles que des polymères ou des macromolécules à activité biologique, notamment de l'ADN, ou des protéines. La présente invention concerne également l'application de cette méthode dans des procédés de mise en évidence, de mesure de distance intra moléculaire, de séparation et/ou de dosage d'une macromolécule dans un échantillon.

Contrôler la conformation de macromolécules représente un enjeu industriel important, par exemple dans la fabrication de capteurs ou d'assemblages moléculaires contrôlés ou encore dans les problèmes de détection et d'analyse. Il peut être intéressant d'avoir une conformation moléculaire allongée. A titre d'exemple, dans le cas où des polymères sont greffés sur un substrat, il a été proposé de les étendre par l'action d'un champ électrique, d'un écoulement ou à l'aide de pinces optiques. En particulier, en biologie, l'alignement de l'ADN - par électrophorèse (Zimmerman et Cox Nucl. Acid Res. 22, p 492, 1994), écoulement libre (Parra et Windle, Nature Genetics, 5, p 17, 1993 et WO 93/22463) ou dans un gel (Schwartz et al. Science 262, p 110, 1993 et USP 33531) ou à l'aide de pinces optiques (Perkins et al., Science 264 p 819, 1994 et aussi USP 5079169) - ouvre de nombreuses possibilités en cartographie, ou dans la détection de pathogènes.

Ces méthodes ne permettent en général qu'un alignement imparfait, ou encore transitoire - c'est-à-dire qu'on a relaxation de la molécule, une fois la contrainte disparue. Dans le cas des pinces optiques, la méthode est lourde, limitée à une seule molécule à la fois, et délicate à mettre en oeuvre par des personnels non qualifiés.

Il a été proposé (I. Parra et B. Windle et WO 93/22463) une technique particulière d'alignement de l'ADN par écoulement après lyse de cellule, puis séchage. L'alignement obtenu est très imparfait et inhomogène et de nombreux amas non alignés sont observés.

La présente invention a pour objet une méthode originale et simple pour aligner des macromolécules sur la surface S d'un support caractérisée en ce que l'on fait se déplacer sur ladite surface S la ligne triple S/A/B (ménisque) résultant du contact d'un solvant A avec la surface S et un milieu B, lesdites macromolécules ayant une partie, notamment une extrémité, ancrée sur la surface S, l'autre partie, notamment l'autre extrémité, étant en solution dans le solvant A.

On a observé selon la présente invention, que le seul passage d'un ménisque sur des molécules dont une partie est ancrée sur un substrat, le reste de la molécule étant librement en solution permet de les aligner uniformément perpendiculairement au ménisque en mouvement les laissant adsorbées sur la surface derrière le ménisque. Ce phénomène est appelé ici "peignage moléculaire".

Plus précisément, l'étirement de la partie libre de la molécule se fait par le passage de la ligne triple S/A/B, constituant le ménisque entre la surface S, le solvant A et un milieu B qui peut être un gaz (en général de l'air) ou un autre solvant.

Dans un mode de réalisation particulier, le ménisque est un ménisque eau-air, c'est-à-dire que le solvant A est une solution aqueuse et le milieu B est de l'air.

De plus il est possible d'étendre le ménisque air/eau utilisé ici afin d'étirer la molécule à d'autres systèmes tels que huile/eau ou eau/surfactant/air, notamment.

Le déplacement du ménisque peut se faire par tout moyen de déplacement relatif des fluides A et B par rapport à la surface S. Dans un mode de réalisation, la surface S peut être retirée du solvant A ou inversement, le solvant A peut être retiré de la surface S.

En particulier le ménisque peut être déplacé à l'aide d'un moyen mécanique, pneumatique notamment en aspirant ou soufflant un gaz, ou hydraulique notamment en poussant ou en aspirant le solvant A ou le milieu B.

Ainsi le déplacement du ménisque peut se faire par évaporation progressive du solvant A.

Lorsque le déplacement du ménisque se fait par voie mécanique, il peut se faire soit par translation de l'interface A/B, soit par translation de la surface S.

Dans un mode de réalisation particulier, le solvant est placé entre deux supports dont un au moins correspond audit support de surface S et le ménisque est déplacé par exemple par évaporation.

On entend ici par "support", tout substrat dont la cohésion est suffisante pour résister au passage du ménisque.

Le support peut être constitué au moins en surface, par un polymère organique ou inorganique, un métal notamment de l'or, un oxyde ou sulfure de métal, un élément semi-conducteur ou un oxyde d'élément semi-conducteur, tel qu'un oxyde de silicium ou une combinaison de ceux-ci, telle que du verre ou une céramique.

On cite plus particulièrement le verre, le silicium oxydé en surface, le graphite, le mica et le sulfure de molybdène.

A titre de "support", on peut utiliser un support unique tel qu'une lame, des billes, notamment de polymère, mais aussi des formes quelconques telles que barre, fibre ou support structuré, et également des particules, qu'il s'agisse de poudres, notamment de poudres de silice, lesquelles peuvent d'ailleurs être rendues magnétiques fluorescentes ou colorées comme cela est connu dans les différentes technologies de dosage.

Le support est avantageusement sous forme de plaques. De préférence, le support ne présente que peu ou pas de fluorescence.

Des macromolécules, telles que polymères quelconques, ou polymères biologiques tels que ADN, ARN ou Protéines, peuvent être ancrés par des méthodes quelconques sur un support.

La macromolécule à aligner peut être choisie parmi les macromolécules biologiques telles que les protéines, notamment les anticorps, antigènes, ligands ou leurs récepteurs, les acides nucléiques, ADN, ARN ou PNA, les lipides, les polysaccharides et leurs dérivés.

On a observé selon la présente invention, que la force d'étirement agit localement au voisinage immédiat du ménisque. Elle est indépendante de la longueur de la molécule, du nombre de molécules ancrées, et dans une large gamme, de la vitesse du ménisque. Ces caractéristiques sont particulièrement intéressantes pour aligner les molécules de façon homogène et reproductible.

On peut, selon la présente invention, ajouter des éléments tensio-actifs dans le solvant A et/ou le milieu B, qui viennent modifier les propriétés des interfaces. Selon la présente invention l'étirement peut en effet être contrôlé par l'addition de tensio-actifs, ou par un traitement de surface adéquat.

Une trop grande attraction surface-macromolécule (par exemple un niveau trop élevé d'adsorption) peut gêner l'alignement des molécules par le ménisque, celles-ci restant adsorbées à la surface dans un état pas nécessairement étiré. De préférence, la surface présente un faible taux d'adsorption de ladite macromolécule, de sorte que seules les molécules ancrées seront alignées, les autres étant entraînées par le ménisque.

Cependant, on peut jouer sur les différences d'adsorption entre une partie de la macromolécule notamment ses extrémités et ses autres parties (en particulier pour de longues molécules, telles l'ADN ou le collagène) pour ancrer par adsorption les molécules par une partie notamment leur(s) extrémité(s) seulement, le reste de la molécule étant librement en solution, sur une très grande variété de surfaces et les aligner par passage du ménisque comme décrit précédemment.

L'adsorption d'une macromolécule sur une surface peut se contrôler aisément à l'aide du pH ou de la teneur du milieu ionique du milieu ou d'une tension électrique appliquée sur la surface. On change ainsi les charges surfaciques et les interactions électrostatiques (répulsives ou attractives) entre la surface et la molécule, ce qui permet de passer d'un état d'adsorption complet de la molécule sur la surface à une absence totale d'adsorption. Entre ces deux cas extrêmes, il existe une plage des paramètres de contrôle où l'adsorption se fait préférentiellement par l'extrémité des molécules et que l'on utilisera donc, avec avantage, pour les ancrer à la surface, puis les aligner par le passage du ménisque.

Les molécules, une fois alignées, adhèrent fortement à la surface. Dans le cas de l'ADN, elles ont pu être observées par fluorescence, plusieurs mois après leur alignement.

La présente invention est donc très différente de la méthode proposée par Parra et Windle, car selon la présente invention, les molécules sont ancrées à la surface puis uniformément alignées par le passage du ménisque, alors que dans la méthode de Parra et Windle un écoulement hydrodynamique est utilisé pour étirer de façon inhomogène les molécules qui vont s'adsorber non-spécifiquement à la surface.

D'autres techniques peuvent aussi conduire à l'étirement et l'alignement de molécules. Ainsi une orientation dynamique de molécules en solution ancrées à une extrémité, peut être obtenue par électrophorèse ou par un écoulement hydraulique.Toutefois, les résultats observés montrent que ces techniques, sont beaucoup moins performantes que l'utilisation du ménisque.

Par "ancrage" de la macromolécule sur la surface il faut entendre une fixation résultant d'une réactivité chimique aussi bien par un lien covalent que par un lien non covalent tel qu'une liaison résultant d'interactions physico-chimiques, tel l'adsorption comme décrit ci-dessus.

Cet ancrage de la macromolécule peut se faire directement sur (ou avec) la surface, ou indirectement, c'est-à-dire par l'intermédiaire d'un lien tel qu'une autre molécule, notamment une autre molécule à activité biologique. Lorsque l'ancrage se fait de manière indirecte, la macromolécule peut être greffée chimiquement sur ledit lien, ou interagir de manière physico-chimique avec ledit lien, en particulier lorsque ledit lien intermédiaire est une molécule à activité biologique reconnaissant et interagissant avec ladite macromolécule.

Dans un mode de réalisation, la macromolécule et ledit lien sont toutes deux des molécules à activité biologique qui interagissent telles qu'antigène et anticorps respectivement, acides nucléiques complémentaires ou lipides. Dans ces cas, la fixation non covalente de la macromolécule consiste en une liaison de type antigène-anticorps, ligand-récepteur, hybridation entre fragments d'acides nucléiques complémentaires ou interaction hydrophobe ou hydrophile entre lipides.

On met ainsi à profit la très grande spécificité et la très grande sélectivité de certaines réactions biologiques, notamment les réactions antigènes/anticorps, les réactions d'hybridation d'ADN ou d'ARN, les réactions interprotéines ou de type avidine/streptavidine/biotine, de même que les réactions des ligands et de leurs récepteurs.

Ainsi, pour réaliser l'ancrage direct ou indirect de la macromolécule sur la surface S on peut utiliser une surface solide présentant certaines spécificités. Il est en particulier possible d'utiliser certaines surfaces prétraitées permettant de fixer certaines protéines ou de l'ADN, qu'il ait été ou non modifié.

De telles surfaces sont commercialement disponibles (Covalink, Costar, Estapor, Bangs, Dynal par exemple) sous différentes formes présentant à leur suface des groupements COOH, NH₂ ou OH par exemple.

On peut alors fonctionnaliser l'ADN avec un groupement réactif, aminé par exemple, et procéder à une réaction avec ces surfaces. Ces méthodes nécessitent cependant une fonctionnalisation particulière de l'ADN à fixer.

On a également décrit une technique permettant l'ancrage sans traitement préalable de l'ADN. Ce procédé consiste à faire réagir un phosphate libre de l'extrémité 5' de la molécule d'ADN avec une amine secondaire de la surface (surface NH Covalink).

L'ancrage par adsorption peut se faire par adsorption de l'extrémité de la molécule en contrôlant la charge surfacique à l'aide du pH, de la teneur ionique du milieu ou de l'application d'une tension électrique sur la surface compte-tenu des différences d'adsorption entre les extrémités de la molécule et sa partie intermédiaire. Selon la présente invention, on a ainsi ancré à titre d'exemple des molécules d'ADN non fonctionnalisées sur des surfaces recouvertes de molécules terminées par un groupe vinyl ou amine telles que des molécules de polylysine ou des surfaces diverses telles que du verre, recouvertes de molécules du type silane terminées par des groupements vinyl ou amine ou encore des lamelles de verre préalablement nettoyées dans un bain d'acide. Dans ce dernier cas, la surface du verre présente en fait des groupes SiOH.

Dans tous ces cas, la plage de pH où l'ADN est ancré est choisie pour être entre un état d'adsorption complet et une absence d'adsorption, cette dernière se situant à pH plus basique. Il est entendu que cette technique est très générale et peut être étendue par l'homme de l'art à de très nombreux types de surfaces.

On peut aussi fonctionnaliser l'ADN avec un premier groupement réactif ou une protéine P₀ pour la faire réagir avec une surface recouverte d'un deuxième groupement réactif ou d'une protéine P₁, susceptibles de réagir spécifiquement entre eux (ou elles) respectivement, c'est-à-dire par exemple, P₁ avec P₀. Le couple P₀/P₁ peut être un couple de type biotine/streptavidine (Zimmermann et Cox) ou digoxigénine/anticorps dirigé contre la digoxigénine (anti-DIG), par exemple (Smith et al., Science 258, 1122 (1992)).

De préférence, les surfaces d'ancrage présenteront un bas taux de fluorescence pour ne pas gêner la détection des molécules après leur alignement, en particulier si celle-ci se fait par fluorescence.

Selon la présente invention, on utilisera de préférence un support solide présentant dans les conditions de réaction une surface ayant une affinité pour une partie de la macromolécule seulement, le reste de celle-ci restant librement en solution.

Dans un mode de réalisation, on utilise un support solide présentant en surface au moins une couche d'un composé organique présentant, à l'extérieur de la couche, un groupement exposé ayant une affinité pour un type de molécule à activité biologique qui peut être ladite macromolécule elle-même ou une molécule reconnaissant et/ou interagissant avec elle.

Le support peut donc présenter une surface recouverte d'un groupement réactif ou d'une molécule à activité biologique.

Par "affinité", il faut entendre ici aussi bien une réactivité chimique qu'une adsorption d'un type quelconque, ceci dans les conditions éventuelles de fixation des molécules sur le groupement exposé modifié ou non.

Dans un mode de réalisation, la surface est essentiellement compacte, c'est-à-dire qu'elle limite l'accès de la macromolécule à activité biologique aux couches inférieures et/ou au support, ceci afin de minimiser les interactions non-spécifiques.

On peut aussi utiliser des surfaces recouvertes d'un groupement exposé réactif (par exemple NH₂, COOH, OH, CHO) ou d'une macromolécule à activité biologique (par exemple : des proteines, telles la streptavidine ou des anticorps, des acides nucléiques tels des oligonucléotides, des lipides des polysaccharides et leurs dérivés) capable de fixer une partie éventuellement modifiée de la molécule.

Ainsi des surfaces recouvertes de streptavidine ou d'un anticorps suivant des procédés connus ("Chemistry of Protein Conjugation and Cross-linking", S.C. Wong, CRC Press (1991)) sont capables de fixer une macromolécule présentant en un site particulier une biotine ou un antigène.

De même des surfaces traitées de manière à présenter des oligonucléotides simple-brin peuvent servir pour y ancrer des ADN/ARN possédant une séquence complémentaire.

Parmi les surfaces comportant un groupement réactif exposé, on cite celles sur lesquelles le groupement exposé est un groupement -COOH, -CHO, NH₂, -OH, ou un groupement vinyl comportant une double liaison -CH=CH₂ utilisée telle quelle ou qui peut être activée pour donner notamment les groupes -CHO, -COOH, -NH₂ ou OH.

Les supports à surfaces hautement spécifiques selon la présente invention peuvent être obtenues par la mise en oeuvre de divers procédés. On peut citer à titre d'exemple :
(A) une couche de polymère carboné, éventuellement branché, d'au moins 1 nm d'épaisseur présentant des groupements réactifs tels que définis ci-après et
(B) des surfaces obtenues par dépôt ou ancrage sur un support solide d'une ou plusieurs couches moléculaires, celles-ci peuvent être obtenues par la formation de couches successives fixées par liaisons non-covalentes, à type d'exemple non limitatif, les films de Langmuir-Blodgett, ou par auto-assemblage moléculaire, ceci permettant la formation d'une couche fixée par liaison covalente.

Dans le premier cas, la surface peut être obtenue par polymérisation d'au moins un monomère générant en surface du polymère ledit groupement exposé, ou bien par dépolymérisation partielle de la surface d'un polymère pour générer ledit groupement exposé, ou encore par dépôt de polymère.

Dans ce procédé, le polymère formé présente des liaisons vyniles tel un dérivé polyénique, notamment des surfaces de type caoutchouc synthétique, tel que le polybutadiène, le polyisoprène ou le caoutchouc naturel.

Dans le deuxième cas, la surface hautement spécifique comporte :
- sur un support, une couche sensiblement monomoléculaire d'un composé organique de structure allongée ayant au moins :
   . un groupement de fixation présentant une affinité pour le support, et
   . un groupement exposé n'ayant pas ou peu d'affinité pour ledit support et ledit groupement de fixation dans les conditions de fixation, mais présentant éventuellement, après une modification chimique suivant la fixation, une affinité pour un type de molécule biologique.

La fixation peut être tout d'abord de type non-covalent, notamment de type hydrophile/hydrophile et hydrophobe/hydrophobe, comme dans les films de Langmuir-Blodgett (K.B. Blodgett, J. Am. Chem. Soc. 57,1007 (1935).

Dans ce cas, le groupement exposé ou le groupement de fixation seront, soit hydrophiles, soit hydrophobes, notamment des groupements alkyles ou halogénoalkyles tels que CH₃, CF₃, CHF₃, CH₂F, l'autre groupement étant hydrophile.

La fixation peut être également de type covalent, le groupement de fixation va alors réagir chimiquement sur le support.

Certaines surfaces de structure approchante ont déjà été mentionnées dans le domaine électronique, notamment lorsque les fixations sont covalentes, L Netzer et J. Sagiv, J. Am. Chem. Soc. 105, 674 (1983) et US-A-4 539 061.

Parmi les groupements de fixation, il faut citer plus particulièrement les groupements de type alkoxyde de métal ou de semiconducteur, par exemple silane, notamment chlorosilane, silanol, méthoxy et éthoxysilane, silazane, ainsi que les groupements phosphate, hydroxy, hydrazide, hydrazine, amine, amide, diazonium, pyridine, sulfate, sulfonique, carboxylique, boronique, halogène, halogénure d'acide, aldéhyde.

Tout particulièrement, comme groupement de fixation on préfèrera utiliser des groupements susceptibles de réagir transversalement avec un groupe équivalent, voisin, pour fournir les liaisons transversales, par exemple il s'agira de dérivés de type alkoxyde de métal ou de semiconducteur, par exemple silane, notamment dichlorosilane, trichlorosilane, diméthoxysilane ou diéthoxysilane et triméthoxy ou triéthoxysilane.

Le choix du groupement de fixation dépendra évidemment de la nature du support, les groupements de type silane sont bien adaptés pour la fixation covalente sur le verre et la silice.

Pour ce qui concerne les groupements exposés, et quelle que soit la surface, ils seront choisis de préférence parmi les groupements éthyléniques, acétyléniques ou des radicaux aromatiques, les amines primaires, tertiaires ou secondaires, les esters, les nitriles, les aldéhydes, les halogènes. Mais il pourra s'agir tout particulièrement du groupement vinyl ; en effet, celui-ci peut être soit modifié chimiquement après fixation pour conduire, par exemple, à un groupement carboxylique ou des dérivés de groupements carboxyliques tels que des groupements alcools, aldéhydes, cétones, acides, amines primaires, secondaires où tertiaires, soit conduire à un ancrage direct pH dépendant des macromolécules biologiques telles que acides nucléiques et protéines sans modification chimique de la surface ou des macromolécules.

De préférence, les chaînes reliant le groupement exposé au groupement de fixation sont des chaînes comportant au moins 1 atome de carbone, de préférence plus de 6 et en général de 3 à 30 atomes de carbone.

Pour ce qui concerne le support lui-même, on préfère utiliser de façon générale, du verre, du silicium oxydé en surface, un polymère ou de l'or avec ou sans prétraitement de la surface.

On peut avantageusement utiliser, dans le cas du verre ou de la silice, les techniques connues de fonctionnalisation de surface utilisant des dérivés silanes, par exemple : Si-OH + Cl₃-Si-R-CH=CH₂ donne Si-O-Si-R-CH=CH₂, R consistant par exemple en (CH₂)₄. Une telle réaction est connue dans la littérature, avec utilisation de solvant ultra-purs. La réaction conduit à un tapis de molécules présentant leur extrémité C=C à la surface exposée à l'extérieur.

Dans le cas de l'or, celui-ci étant éventuellement sous la forme d'une couche mince sur un substrat, les techniques connues de fonctionnalisation de surface utilisent des dérivés thiols, par exemple : Au + HS-R-CH=CH₂ donne Au-S-R-CH=CH₂, R consistant par exemple en (CH₂)₄. Une telle réaction est décrite en milieu liquide et conduit, de même que la réaction précédente trichlorosilane-silice, à un tapis de molécules présentant leur extrémité C=C à la surface exposée à l'extérieur.

Bien entendu la terminologie de "support" englobe aussi bien une surface unique telle qu'une lame, mais également des particules qu'il s'agisse de poudre de silice ou de billes de polymère, et aussi des formes quelconques telles que barre, fibre ou support structuré, lesquelles peuvent d'ailleurs être rendues magnétiques, fluorescentes ou colorées, comme cela est connu dans différentes technologies de dosage.

De préférence, le support sera choisi pour être pas ou peu fluorescent lorsque la détection sera effectuée par fluorescence.

Les surfaces obtenues selon les modes (A) ou (B) ci-dessus présentent :
(i) un très faible taux de fluorescence intrinsèque, lorsque cela est requis, un bruit de fond de fluorescence (d'une aire typique de 100 x 100 µm) plus faible que le signal de fluorescence d'une seule molécule à détecter ;
(ii) la possibilité de détecter des molécules isolées avec un rapport S/N indépendant du nombre de molécules, qui est possible grâce à différentes techniques à grand rapport S/N décrites plus bas et basées sur l'identification de la présence d'un marqueur macroscopique présentant une faible interaction non-spécifique avec la surface.

Les surfaces ainsi obtenues sont, de préférence, revêtues d'une macromolécule à activité biologique choisie parmi :
- les protéines,
- les acides nucléiques,
- les lipides,
- les polysaccharides et leurs dérivés.

Parmi les protéines, il faut citer les antigènes et les anticorps, les ligands, les récepteurs, mais également des produits de type avidine ou streptavidine ainsi que les dérivés de ces composés.

Parmi les ARN et les ADN, il faut également citer les dérivés α, β ainsi que les dérivés thio et les composés mixtes tels que les PNA.

On peut également fixer des composés mixtes tels que les glycopeptides et les lipopolysaccharides par exemple, ou bien d'autres éléments tels que virus, cellules notamment, ou composés chimiques tels que la biotine.

La fixation des macromolécules biologiques peut être covalente ou non-covalente, par exemple par adsorption, liaisons hydrogènes, interactions hydrophobes, ioniques, par exemple, auquel cas on pourra procéder avantageusement à un pontage ("cross-linking") entre les molécules greffées par les méthodes connues ("Chemistry of Protein Conjugation and Cross-linking", S.C. Wong, CRC Press (1991)) et ceci afin de renforcer leur cohésion.

Comme cela a été mentionné précédemment, il est possible d'avoir un groupement exposé qui permet la réaction directe avec les molécules à activité biologique, mais il est également possible de prévoir que le groupement exposé est traité, après fixation, pour être transformé, comme cela a été indiqué précédemment, en un radical hydroxy, amine, alcool, aldéhyde, cétone, COOH ou dérivé de ces groupements avant la fixation de la molécule biologique.

Lorsque de tels groupements ont été exposés, les techniques de fixation des protéines et/ou de l'ADN, par exemple, sont connues, il s'agit en effet de réactions mises en oeuvre pour des surfaces qui sont déjà utilisées dans le cadre des analyses biologiques, notamment pour les surfaces Costar et les surfaces Nunc ou des microbilles telles qu'Estapor, Bang et Dynal par exemple, sur lesquelles on ancre des molécules d'intérêt biologique, ADN, ARN, PNA, protéines ou anticorps par exemple.

Dans le cas où le groupement exposé est un radical -CH=CH₂ qui est nommé ci-après "surface C=C" ou "surface à liaison éthylénique", il n'existe pas de document mentionnant l'ancrage direct, en particulier de l'ADN ou des protéines.

Dans le cadre de la présente invention, il a été démontré que ces surfaces présentent une réactivité fortement pH dépendante. Cette particularité permet d'ancrer les acides nucléiques ou les protéines en utilisant des zones de pH et souvent avec une vitesse de réaction qui peut être contrôlée par le pH.

On peut réaliser l'ancrage de l'ADN par son extrémité sur une surface présentant des groupements à double liaison éthylénique en mettant l'ADN en présence de la surface à un pH inférieur à 8.

En particulier la réaction est conduite à un pH compris entre 5 et 6 puis est stoppée à pH 8.

Ainsi, pour l'ADN à pH 5,5, la réaction d'ancrage est totale en une heure (si pas limitée par la diffusion) et se produit par les extrémités. A pH 8, par contre, l'accrochage est très faible (vitesse de réaction de 5 à 6 ordres de grandeur plus faibles). Cet effet d'accrochage pH dépendant et spécifique des extrémités, présente une amélioration par rapport aux autres surfaces qui nécessitent une fonctionnalisation de l'ADN (biotine, DIG, NHS, ...) ou des réactifs spécifiques (carbodiimide, diméthyle pimélidate) qui réalisent une liaison peptidique ou phosphorimide entre -NH₂ et -COOH ou -POOH.

On peut aussi réaliser l'ancrage d'ADN par adsorption de ses extrémités seulement sur une surface recouverte de polylysine ou d'un groupement silane terminé par un groupe amine.

Pour réaliser l'ancrage de l'ADN par son extrémité sur une surface recouverte par un groupement amine on met l'ADN en présence de la surface à pH entre 8 et 10.

De même on peut réaliser l'ancrage d'ADN par son extrémité sur une surface de verre traité auparavant dans un bain d'acide, en mettant l'ADN en présence de ladite surface à pH entre 5 et 8.

Il va de soi que la présente invention implique, dans le même esprit, l'accrochage éventuellement pH dépendant de toutes macromolécules d'intérêt biologique.

De même ces surfaces peuvent ancrer des protéines directement (protéine A, anti-DIG, anticorps, streptavidine, etc.). Il a été observé que (i) l'activité de la molécule peut être préservée et (ii) que la réactivité de la surface préparée (initialement C=C) est totalement occultée pour faire place à la seule réactivité de la molécule d'intérêt. Il est donc possible, à partir d'une réactivité initiale relativement large, de passer à une surface possédant une réactivité très hautement spécifique, par exemple celle de sites spécifiques sur une protéine.

En ancrant un anticorps spécifique sur la surface (par exemple anti-DIG), on crée une surface dont la réactivité est limitée à l'antigène (par exemple le groupement DIG). Ceci indique que les groupements chimiques initiaux ont tous été occultés par les anticorps greffés.

On peut aussi greffer sur les surfaces réactives (chimiquement ou biochimiquement) d'autres molécules à activité biologique, notamment des virus ou d'autres composants : membranes, récepteurs membranaires, polysaccharides, PNA, notamment.

Il est également possible de fixer le produit d'une réaction d'intérêt biologique (par exemple la PCR) sur les surfaces préparées.

Le procédé selon la présente invention permet la mise en évidence et/ou la quantification de molécules biologiques, mais également la mesure de distance intramoléculaire, la séparation de certaines molécules biologiques, notamment un prélèvement par mise en oeuvre des techniques de couplage antigène/anticorps et/ou ADN/ARN.

En particulier, la présente invention a pour objet un procédé de mise en évidence d'une macromolécule consistant en une séquence d'ADN ou d'une protéine dans un échantillon, selon la présente invention, caractérisé en ce que :
- on met l'échantillon correspondant au solvant A, dans lequel ladite macromolécule est en solution, en contact avec la surface du support dans des conditions de formation d'un hybride ADN/ADN, ADN/ARN ou de formation du produit de réaction protéine/protéine,
- l'hybride ou une macromolécule de marquage de l'hybride ou du produit de réaction étant ancré en une partie, le reste étant libre en solution, on l'étire par déplacement du ménisque créé par le contact du solvant avec la surface pour orienter les hybrides ou lesdites macromolécules de marquage et on effectue la mesure ou l'observation des hybrides ou desdites macromolécules de marquage ainsi orientés.

Avantageusement, l'ADN fixé et l'ADN de l'échantillon sont colorés de façon différente et après étirement on mesure la position de la séquence complémentaire par rapport à l'extrémité de l'ADN de l'échantillon.

De façon appropriée on peut utiliser les méthodes de détection ELISA ou FISH.

L'échantillon d'ADN peut être le produit ou le substrat d'une amplification enzymatique d'ADN telle que la PCR, c'est-à-dire qu'on peut réaliser l'amplification de l'ADN une fois celui-ci ancré et aligné selon le procédé de l'invention ou avant son ancrage et son alignement.

Le passage du ménisque, en étirant linéairement les molécules, sous forme de bâtonnets, les rend plus facilement détectables si elles sont marquées. Par ailleurs, ces molécules allongées sont stables à l'air libre et peuvent être observées même après plusieurs mois, sans présenter de dégradation apparente.

Lors d'une réhydratation, les molécules d'ADN peuvent rester adsorbées et allongées. De plus, il est possible de procéder à une hybridation sur la molécule allongée.

De plus, présentant un signal corrélé et d'orientation uniforme de par leur étirement, ces molécules sont distinctes du bruit environnant. Il est donc facile d'ignorer les poussières, les inhomogénéités, qui ne présentent pas de corrélation spatiale particulière. L'alignement est aussi intéressant car en solution, les molécules en pelote fluctuent thermiquement, ce qui entraîne des variations très importantes de leur signal de fluorescence recueilli de préférence avec une faible profondeur de champs et limite leur observation. La présente invention permet donc l'observation de molécules isolées avec un très grand rapport signal sur bruit. (S/N).

Il est remarquable que ce rapport soit indépendant du nombre de molécules ancrées. Le rapport S/N posé par la détection d'une molécule est le même que pour 10 000. De plus cette technique d'étirement permet de discriminer aisément entre des molécules de longueurs variées.

On peut avantageusement procéder aux étapes suivantes pour améliorer encore le rapport S/N :
- La molécule étant immobile, on peut intégrer son signal de fluorescence.
- L'observation au microscope présente un champ réduit (typiquement 100 µm x 100 µm avec un objectif x 100 à immersion, N.A. = 1.25). Pour un échantillon de 1 cm² on peut soit procéder à un balayage, soit envisager l'utilisation d'objectifs d'agrandissements moindres (x 10 ou x 20) mais d'ouverture numérique élevée.
- Les bâtonnets étant toujours parallèles, on peut envisager une méthode de filtrage spatial optique pour augmenter encore le rapport S/N.
- D'autres méthodes de fluorescence globale sont envisageables telles que celles décrites dans la demande de brevet européen EP 103426.
- La linéarisation des molécules s'observe aussi bien dans le cadre d'un ancrage physico-chimique que dans le cas de liaisons de type immunologique (DIG/anti-DIG).
- Une fois la surface à l'air libre, les molécules d'ADN sont stables (restent intègres, même après plusieurs semaines) et fluorescentes. On peut avantageusement utiliser cette propriété pour différer l'étape d'ancrage de l'étape de repérage/comptage des molécules ancrées, si cette détection se fait par exemple, mais sans s'y limiter, par microscopie à fluorescence. Une telle utilisation est couverte par la présente invention.
- Une technique de double (ou multi) fluorescence peut éventuellement servir à améliorer le rapport S/N ou à détecter une double fonctionnalité.

Les molécules étirées peuvent être révélées par différentes méthodes enzymologiques ou autres, telles la fluorescence ou l'utilisation de sondes chaudes ou froides. Leur détection peut se faire par mesure d'un signal global (par exemple la fluorescence) ou par l'observation individuelle des molécules par microscopie optique à fluorescence, microscopie électronique, méthodes à sondes locales (STM, AFM, etc.).

Ainsi de façon générale, la présente invention permet la mise en évidence, la séparation et/ou le dosage d'une molécule dans un échantillon, par un procédé caractérisé en ce qu'on utilise une surface capable de fixer spécifiquement ladite molécule, et en ce que la mise en évidence, la séparation ou le dosage sont effectués grâce à un réactif fluorescent ou non détectant la présence de la molécule fixée.

Parmi les réactifs on distingue les réactifs fluorescents et les réactifs non-fluorescents.

Les réactifs fluorescents contiennent des molécules fluorescentes, choisies avec avantage pour être des molécules longues de taille supérieure à 0,1 µm et réagissant de manière spécifique directement ou indirectement avec les surfaces prétraitées. Par exemple, mais sans pour autant s'y limiter, une molécule d'ADN double brin teintée à l'aide de sondes fluorescentes (ethidium bromide, YOYO, nucléotides fluorescents, etc.) pouvant s'ancrer directement par une ou plusieurs extrémités sur une surface présentant éventuellement un groupement de type vinyl, amine ou autre. notamment par un choix judicieux du pH ou de la teneur ionique du milieu ou par application d'une tension électrique sur la surface.

Il est également possible d'utiliser une fonctionnalisation particulière de la molécule (DIG, biotine etc...) pour l'ancrer en un ou plusieurs points sur une surface présentant des sites complémentaires (anti-DIG, streptavidine, etc...).

Les réactifs non-fluorescents permettant la détection de molécules préalablement alignées selon la présente invention, peuvent consister notamment en des billes ou microparticules ancrées par l'intermédiaire d'une autre molécule fixée de manière spécifique directement ou indirectement à la molécule alignée et ne présentant qu'une faible interaction non-spécifique avec la surface.

Par exemple, on peut citer des billes Dynal recouvertes de streptavidine permettant l'ancrage sur ADN biotynilé, aligné selon la présente invention.

Selon que la molécule recherchée est détectée directement par fluorescence ou indirectement à l'aide des réactifs ci-dessus, on parlera de "détection directe" ou "par drapeau".

Afin de limiter les problèmes associés à des temps de réaction trop lents, on peut avantageusement réduire les temps de diffusion des réactifs vers la surface en utilisant de petits volumes de réaction. Par exemple, mais sans s'y limiter, en conduisant la réaction dans un volume de quelques microlitres déterminé par l'espacement entre deux surfaces dont l'une est traitée pour présenter des sites réactifs et l'autre est inerte ou traitée pour ne pas présenter de sites réactifs, dans les conditions de la réaction.

La détection du nombre de molécules alignées peut être réalisée sur un petit nombre de molécules (typiquement 1 à 1 000), par un test physique macroscopique faible bruit ne nécessitant ni microscope électronique ni radioactivité ni nécessairement la PCR.

Les procédés d'alignement et de détection selon la présente invention sont susceptibles d'être mis en oeuvre par des personnes n'ayant qu'une expérience de laboratoire réduite.

La spécificité de certaines réactions biologiques peut être limitée. Ainsi, dans le cadre de l'hybridation, les hybrides peuvent être imparfaits (réactions avec d'autres sites) tout en présentant un nombre réduit d'appariement et donc une qualité de liaison moindre. La présente invention couvre également l'utilisation possible d'une étape de test de la qualité des liaisons obtenues. Ce test permet de dissocier les produits appariés de façon non-spécifique faible, par adsorption, forces hybrophobes, liaisons hydrogènes imparfaites, hybridation imparfaite, notamment.

C'est pourquoi, l'invention concerne également dans un procédé de mise en évidence ou de dosage tel que décrit précédemment, un procédé où l'on soumet le produit de réaction entre la molécule à activité biologique et la molécule de l'échantillon à une contrainte afin de détruire les mauvais appariements avant la détection.

Ce procédé offre, outre la possibilité de détruire les couples mésappariés, la possibilité d'orienter les produits du couplage, ce qui facilite les mesures ou les observations.

On peut ainsi appliquer aux surfaces, après fixation des éléments complémentaires, une contrainte qui peut être constituée par l'utilisation simple ou combinée de :
- centrifugation,
- gradient de champ magnétique appliqué aux réactifs non-fluorescents pris alors pour inclure des microbilles magnétisables ou magnétiques,
- agitation,
- écoulement liquide,
- passage de ménisque,
- électrophorèse
- variation de température, et/ou gradient de température.

On détermine alors par les techniques de détection faible bruit décrites ci-dessus le nombre de systèmes étant restés intègres ou s'étant détruits.

Les techniques d'alignement et de détection décrits selon la présente invention peuvent être utilisés pour de nombreuses applications parmi lesquelles, mais sans s'y restreindre :
- l'identification d'un ou plusieurs éléments de séquence d'ADN ou d'ARN que l'on peut utiliser avec avantage pour le diagnostic de pathogènes ou la cartographie physique d'un génome. En particulier, les techniques décrites ci-dessus permettent l'obtention d'une cartographie physique directe sur ADN génomique, sans passer par une étape de clonage. Il est entendu que la molécule peignée étant étirée par rapport à sa longeur crystallographique, on procède à des mesure relatives. Il est ainsi possible de mesurer la taille de fragments d'ADN et la distance entre fragments avec une résolution de l'ordre de 200 nm pour des méthodes optiques ou de l'ordre de 1 nm par l'utilisation de méthodes de champs proche telles que AFM ou STM pour visualiser et mesurer la distance entre sondes sur de l'ADN aligné.
   Ceci conduit naturellement à :
   1) la détection de délétions, additions ou translocations de séquences génomiques, en particulier dans le diagnostic de maladies génétiques (par exemple la myopathie de Duchesne) ;
   2) l'identification de promoteurs de différents gènes par la mesure de la distance entre les séquences régulatrices et celle exprimées ;
   3) la localisation de protéines régulatrices par l'identification de leur position le long de l'ADN ou de la position de leur séquence cible ;
   4) le séquençage partiel ou total par mesure de la distance à l'aide de microscopies à champs proche (par exemple AFM ou STM) entre des sondes hybridées appartenant à une base d'oligonucléotides de longueur donnée.
- L'amplification enzymatique in situ sur des ADN alignés.
- l'amélioration de la sensibilité des techniques d'ELISA avec la possibilité de détecter un faible nombre (éventuellement inférieur à 1 000) de réactions immunologiques.

Ainsi, on peut procéder à une cartographie physique directement sur un ADN génomique sans passer par une étape de clonage. L'ADN génomique est extrait, purifié, éventuellement coupé par un ou plusieurs enzymes de restriction puis peigné sur des surfaces selon le procédé de la présente invention.

La position et la taille du gène recherché sur l'ADN génomique sont alors déterminées par hybridation avec des sondes spécifiques dudit gène, notamment extraites de parties de l'ADN complémentaire (cDNA) du produit dudit gène recherché.

De façon similaire en hybridant un ADN génomique peigné, puis dénaturé avec du cDNA total marqué par fluorescence ou tout autre marqueur permettant de localiser l'hybride, on identifie la position, la taille et le nombre des exons du gène en question, d'où l'on déduit sa taille et son organisation génétique (exons, introns, séquences régulatrices).

La position du gène étant déterminée comme décrit ci-dessus, ou connue, il est alors possible d'identifier par hybridation les séquences flanquantes du gène. Pour cela, on procèdera, avec avantage, par hybridation avec des sondes marquées, provenant par exemple d'une librairie d'oligonucléotides, pour identifier deux ou plusieurs sondes qui s'hybrident de part et d'autre du gène.

A partir de cette détermination, il est alors possible par les techniques d'amplification enzymatique, par exemple la PCR in situ (Nuovo G.J. PCR in situ hybridization : protocoles and applications, Raven Press (1992)) d'amplifier le fragment délimité par les sondes flanquantes qui peuvent servir d'amorces à la réaction, fragment qui peut contenir le gène recherché avec ses régions régulatrices qui peuvent être tissu ou développement spécifique et qu'on peut alors isoler et purifier.

On peut aussi procéder par polymérisation in situ sur des amorces extraites du cDNA du gène en question pour extraire des fragments d'ADN complémentaires des régions flanquantes du gène comme mentionné par Mortimer et al. (Yeast 5, 321 1989). Ces fragments peuvent alors servir dans la préparation d'amorces pour un procédé d'amplification enzymatique du géne et de ses séquences flanquantes.

On peut aussi utiliser les méthodes citée par A. Thierry et B. Dujon (Nucl. Acid Research 20 5625 (1992)) pour insérer par recombinaison homologue ou aléatoirement des sites spécifiques connus d'endonucléase dans un ADN génomique ou un fragment d'ADN génomique. Le peignage de cet ADN permet l'identification du gène d'intérêt et des sites spécifiques insérés, par les méthodes d'hybridation in situ décrites ci-dessus. A partir de cette identification et préférablement si les sites d'intérêt sont des régions d'intérêt proches du gène, on les utilisera comme amorce d'une réaction d'amplification enzymatique (in situ ou autre) du gène en question et de ses séquences flanquantes.

L'amplification du gène recherché procède alors par les techniques d'ampilfication enzymatique connues telles que PCR sur le fragment amplifié comme décrit ci-dessus en utilisant des amorces accessibles par les exons constituant le cDNA, ou des amorces correspondant à des séquences flanquantes.

Par le peignage d'ADN génomique ou autre, il est aussi possible de déterminer par hybridation la présence ou l'absence de séquences régulatrices d'un gène particulier proximal, à partir desquelles on déterminera les familles possibles de protéines régulatrices de ce gène (par exemple : helix-loop-helix, zinc-finger, leucine-zipper).

Les réactions spécifiques entre séquences particulières d'ADN/ARN/PNA et une autre molécule (ADN, ARN, protéine) peuvent se faire avant ou après alignement des molécules selon la présente invention.

Ainsi, dans le cadre du diagnostic génétique et de la cartographie physique, on utilise avec avantage les méthodes connues de FISH (Pinkel et al., Proc. Nat. Acad. Sci. USA 83, 2934 (1986)) pour hybrider des oligonucléotides simple-brin marqués à de l'ADN d'abord aligné, puis dénaturé. La révélation des hybrides se fera par les techniques connues (fluorescences, microbilles etc...) avec une résolution dans la mesure des distances allant de 0,2 µm (en optique) à 1nm (en microscopie à champ proche ; AFM, STM etc...).

Alternativement, on peut d'abord hybrider des ADN marqueurs fluorescents à de l'ADN simple-brin en solution, puis aligner cette construction par l'action du ménisque après l'avoir transformé en ADN double-brin et ancré à une surface adéquate.

On peut aussi utiliser la présente invention pour la détection de la présence d'un pathogène. A titre d'exemple, on pourra procéder de deux manières différentes selon que la réaction de reconnaissance (hybridation, attachement de protéine) ait lieu avant ou après alignement par le ménisque.

Ainsi, à titre d'exemple, un ou plusieurs oligonucléotides sondes sont ancrées à une ou plusieurs régions d'une surface. L'hybridation de l'ADN potentiellement pathogénique est effectuée in situ dans des conditions stringentes de façon à n'ancrer que les molécules hybridées. Leur détection et quantification s'effectuent après alignement par le ménisque selon la présente invention.

Alternativement, l'ADN potentiellement pathogénique est d'abord aligné, puis dénaturé et hybridé avec un oligonucléotide sonde dans des conditions stringentes. La détection de l'hybride s'effectue alors par les méthodes connues notamment de FISH, comme décrit ci-dessus.

D'une manière similaire, on peut détecter la présence (ou l'absence) d'un petit nombre de molécules, telles des protéines, des lipides, des sucres ou des antigènes. On procèdera avec avantage, à une modification mineure des techniques d'ELISA, la méthode de détection habituelle étant remplacée par la détection d'une molécule fluorescente alignée selon la présente invention et couplée à l'un des réactifs de la réaction d'ELISA.

Par ailleurs, comme mentionné par K.R. Allan et al. (US 84 114), La cartographie génétique peut procéder par une mesure de la taille de fragments d'ADN. Or les techniques originales d'étirement des molécules décrites plus haut (l'étirement par le ménisque) permet une mesure de la longueur des molécules étirées et cela sur un très petit échantillon (quelques milliers de molécules).

On peut, par exemple, mais sans sy restreindre, procéder de la manière suivante :
Un échantillon d'ADN est fragmenté (à l'aide d'enzymes de restriction), teinté avec un fluorophore puis ancré sur une surface. Les molécules sont ensuite étirées par le ménisque et la taille des fragments étirés déterminée par microscopie optique à fluorescence avec une résolution et une taille limite de l'ordre de 1 000 bp (0,3 µm).

Dans ce but, mais aussi si l'on veut aligner des molécules très longues (≥ 10µm) on utilisera avec avantage des techniques connues pour limiter la dégradation de longues macromolécules lors de leur manipulation (par cisaillement hydrodynamique).

Ainsi comme mentionné par D.C. Schwartz, on procédera avec avantage, à une condensation des molécules à l'aide d'un agent condensateur (par exemple la spermine ou un alcool) lors de leur manipulation. Eventuellement, leur décondensation s'effectuera lors du contact du solvant A avec la surface d'ancrage S.

Afin de réduire la dégradation des macromolécules lors de l'étirement par le ménisque, on utilisera des techniques de translation du ménisque qui minimisent le cisaillement hydrodynamique. Par exemple, mais sans pour autant s'y restreindre, en retirant très lentement (≤200 µ/sec) la surface S, d'un volume conséquent (≥ 100µl) du solvant A.

La présente invention est utile pour obtenir une surface présentant un ou plusieurs types de macromolécules alignées obtenues selon la présente invention. En particulier, on peut obtenir une surface ou un empilement de surfaces présentant des propriétés électriques ou optiques anisotropes.

La présente invention a aussi pour objet un procédé d'alignement et de mise en évidence de l'ADN dans lequel l'ADN est étiré par un procédé d'alignement selon l'invention, puis dénaturé puis hybridé avec des sondes spécifiques pour déterminer la position ou la taille d'une ou plusieurs séquences spécifiques.

La présente invention a également pour objet un procédé de cartographie physique d'un gène sur un ADN génomique dans lequel l'ADN est aligné ou mis en évidence selon un procédé de l'invention.

En particulier, la position et la taille du gène recherché sur l'ADN génomique, sont déterminées par hybridation avec des sondes spécifiques dudit gène à cartographier.

La présente invention est également utile pour préparer
- un coffret utile pour la mise en oeuvre d'un procédé de cartographie selon l'invention, constitué par de l'ADN génomique total d'un hôte de référence,
- un support présentant une surface permettant l'ancrage et l'alignement de l'ADN du patient conformément au procédé de l'invention
- des sondes spécifiques du ou des gène(s) à cartographier et des réactifs pour l'hybridation et la détection de l'ADN.

La présente invention a également pour objet un procédé d'alignement et de mise en évidence de l'ADN dans lequel l'ADN est étiré puis dénaturé puis hybridé avec des sondes spécifiques pour déterminer la présence ou l'absence d'une ou plusieurs séquences d'ADN dans ledit ADN aligné.

La présente invention permet la mise en oeuvre d'un procédé de diagnostic d'une pathologie liée à la présence ou l'absence d'une séquence d'ADN spécifique de la pathologie dans lequel on utilise un procédé d'alignement selon l'invention.

La présente invention est également utile pour préparer un coffret utile pour la mise en oeuvre d'un procédé de diagnostic selon l'invention caractérisé en ce qu'il comporte un support dont la surface permet l'ancrage et l'alignement de l'ADN du patient selon un procédé de l'invention, des sondes spécifiques du gène impliqué dans la pathologie recherchée et des réactifs pour l'hybridation et la détection de l'ADN.

La présente invention est également utile pour préparer un coffret utile pour la mise en oeuvre d'un procédé de diagnostic selon l'invention caractérisé en ce qu'il comporte un support dont la surface présente des sondes spécifiques du gène impliqué dans une pathologie, en particulier de l'ADN pathogène éventuellement marqué, alignées selon le procédé de la présente invention et éventuellement dénaturées ; les réactifs pour préparer et marquer l'ADN du patient en vue de son hybridation (par exemple la photobiotine, kit de "nick-translation" ou de "random priming") et des réactifs pour l'hybridation et la détection de l'ADN suivant les techniques d'hybridation in situ comme décrites ci-dessus.

Il est entendu que des sondes peignées relatives à des pathogènes différents peuvent être présentes sur différents supports ou sur le même support. L'identification du pathogène correspondant peut se faire après hybridation, soit spacialement (les différentes sondes sont séparées spacialement par exemple par ancrage photochimique avant leur peignage) soit par une différence du spectre de fluorescence des différents hybrides, résultant d'un marquage différentiel préalable des sondes.

Enfin, la présente invention a pour objet un procédé de préparation d'un gène dans lequel on identifie la position dudit gène sur de l'ADN génomique aligné par le procédé selon l'invention à l'aide d'une sonde spécifique dudit gène on amplifie par amplification enzymatique, notamment, PCR in situ la séquence dudit gène et éventuellement ses séquences flanquantes.

La présente invention permet donc de mettre en oeuvre un procédé de remplacement d'un gène dans le génome d'une cellule eucaryote par l'insertion ciblée d'un gène étranger à l'aide d'un vecteur contenant ledit gène étranger préparé selon le procédé de préparation de gène ci-dessus.

L'insertion ciblée peut être effectuée selon les techniques décrites dans WO90/11354 en transfectant des cellules eucaryotes avec un vecteur contenant ledit ADN étranger à insérer flanqué de deux séquences génomiques qui jouxtent le site d'insertion souhaité dans le gène receveur. L'ADN d'insertion peut comporter soit une séquence codante, soit une séquence régulatrice. Les séquences flanquantes sont choisies afin de permettre par recombinaison homologue, selon le cas, soit l'expression de la séquence codante de l'ADN d'insertion sous le contrôle des séquences régulatrices du gène receveur, soit l'expression d'une séquence codante du gène receveur sous le contrôle de séquence régulatrice de l'ADN d'insertion.

Les gènes génomiques et les cDNA obtenus en utilisant le procédé de localisation de gènes selon l'invention peuvent être insérés dans des vecteurs d'expression capables de s'insérer dans une cellule hôte procaryote, eucaryote ou virale. Les protéines polypeptides et peptides dérivés sont inclus dans la présente invention.

La description qui va suivre, est faite en se référant aux figures annexées sur lesquelles :
- la figure 1 schématise la détection d'un pathogène dans une molécule d'ADN fluorescente par hybridation avec une molécule ancre ;
- la figure 2 schématise la cartographie génétique par extension de l'ADN et l'utilisation d'un ADN marqueur ;

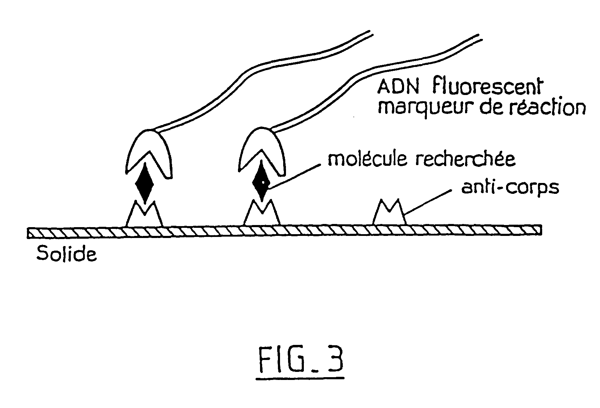

- la figure 3 schématise la détection d'une réaction immunologique (ELISA) à l'aide d'une molécule "drapeau" : un ADN fluorescent utilisé comme marqueur de réaction ;

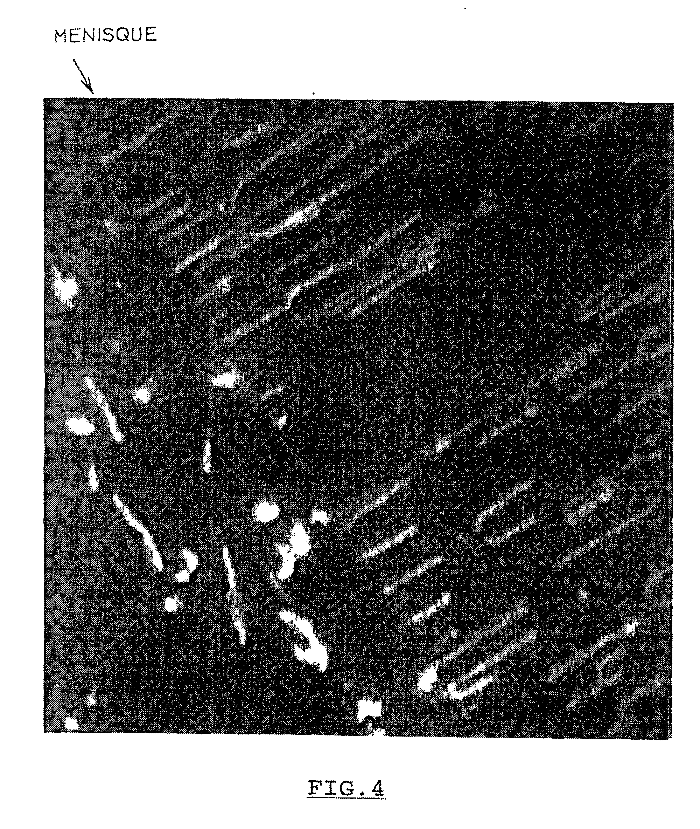

- la figure 4 est une microphotographie de fluorescence montrant l'extension d'ADN de phage λ par l'avancée du ménisque, à gauche on aperçoit des molécules d'ADN en solution étirées par l'écoulement d'évaporation parallèle au ménisque, à droite des molécules d'ADN à l'air libre après leur étirement perpendiculairement au ménisque ;

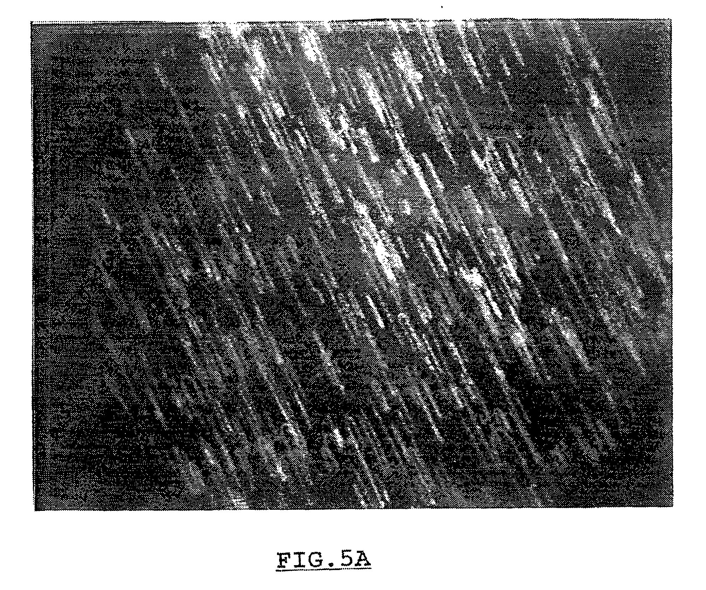

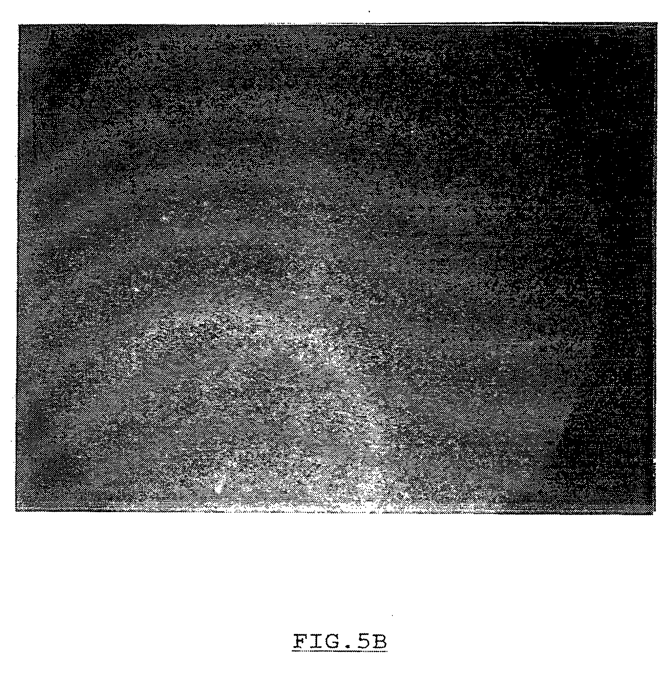

- les figures 5(a) et 5(b) sont des microphotographies de fluorescence montrant, respectivement, un ADN marqué à la digogixénine (DIG) sur une surface recouverte d'anti-DIG et étiré par le ménisque, et, en contrôle, un ADN non marqué sur une surface anti-DIG, on remarquera la très grande spécificité des surfaces et l'absence d'ancrage non-spécifique ;

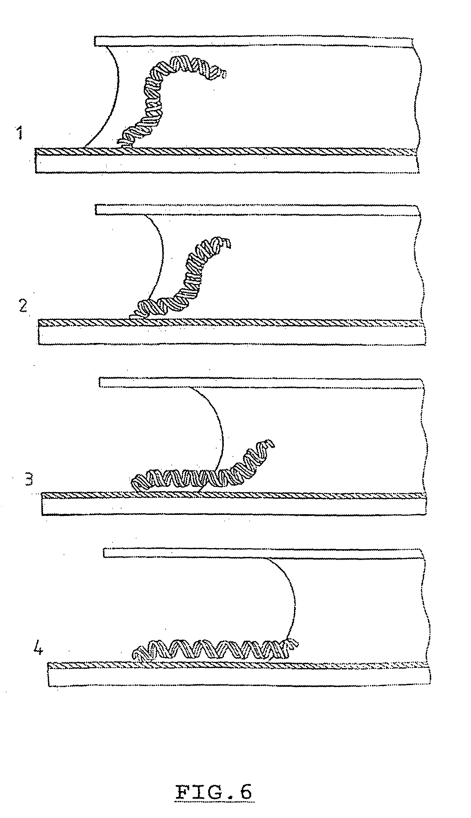

- la figure 6 représente le schéma de l'étalement de l'ADN par passage du ménisque. L'ADN en solution est ancré sur une surface traitée. La solution d'ADN est recouverte d'une lamelle ronde non-traitée ;
- la figure 7 représente des histogrammes de la longueur des molécules d'ADN λ peignées sur des surfaces de verre :
   a) recouvertes de molécules silanes terminées par un groupement amine, b)recouvertes de polylysine,
   c) nettoyées dans un mélange eau oxygénée/acide sulfurique.
- la figure 8 représente des molécules d'ADN peignées sur des surfaces de verre recouvertes de polylysine. On remarque que les molécules attachées par leurs deux extrémités forment des boucles.
- la figure 9 représente des YACs peignés par retrait d'une lamelle traitée d'une solution de ces molécules.
- la figure 10 montre l'identification de la présence et de la taille d'un cosmide sur un YAC par hybridation in situ.

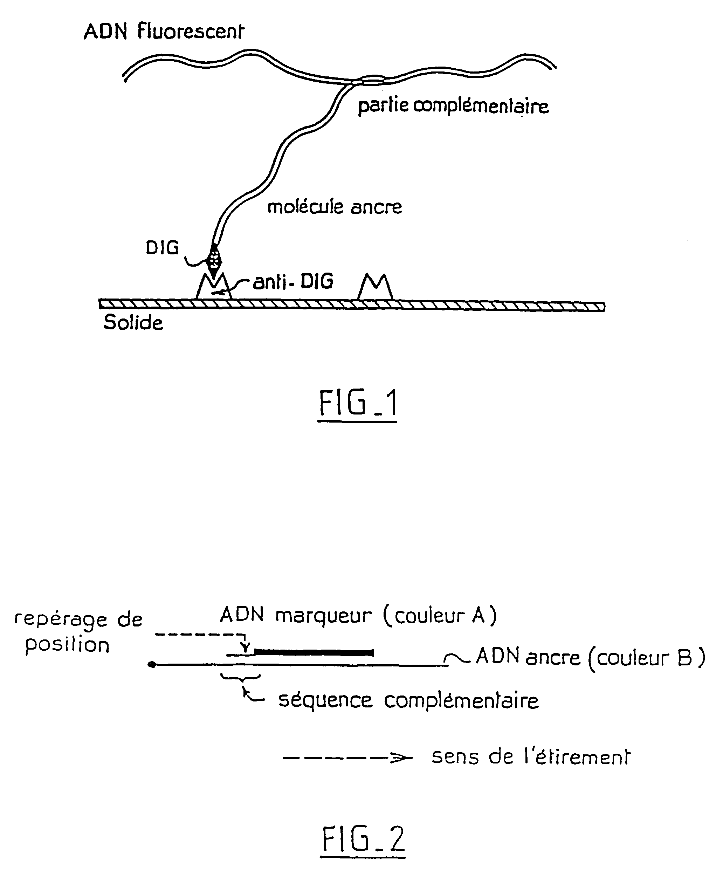

Dans le mode "diagnostic", les sondes (les "ancres") possèdent un groupement réactif (DIG, biotine, etc.) capable de s'ancrer de manière spécifique à une surface selon la présente invention (ayant par exemple comme site d'ancrage un anticorps anti-DIG ou la streptavidine). La détection de la réaction d'ancrage peut se faire directement par détection de la fluorescence de la molécule d'ADN teintée par des molécules fluorescentes (ethidium bromide, YOYO, nucléotides fluorescents) (figure 1). Elle peut aussi se faire indirectement par détection d'une "molécule drapeau" : un réactif capable de se fixer sur la molécule d'ADN/ARN (par exemple par hybridation, interaction protéine-ADN, etc.), mais ne présentant pas d'affinité pour les sites d'ancrage de la sonde.

Dans le mode "cartographie" on peut utiliser les techniques d'hybridation in situ (FISH). Il est aussi possible d'envisager d'autres techniques, par exemple en hybridant en solution de l'ADN avec des sondes présentant des réactifs fluorescents selon la présente invention. La détection de la position des sondes se fait après l'alignement de la molécule selon la présente invention.

### EXEMPLE 1

### Matériels et méthodes

L'ADN-λ et l'anticorps monoclonal (Anti-DIG) proviennent de Boehringer-Mannheim. Les trichlorosilanes proviennent de Roth-Sochiel. Les sondes nucléiques fluorescentes (YOYO1, YOYO3 et POPO1) proviennent de Molecular Probes. Les lamelles de verre ultrapropres proviennent de Erie Scientific (Lamelles (ESCO). Les particules magnétiques proviennent de Dynal. Le microscope est un microscope inversé Diaphot de NIKKON, équipé d'une lampe Xenon pour l'épifluorescence et d'une caméra CCD intensifiée Hamamatsu pour la visualisation.

### Traitement de surface

Des lamelles de verre sont nettoyées pendant une heure par irradiation UV sous atmosphère d'oxygène (par formation d'ozone). Elles sont ensuite immédiatement déposées dans un dessicateur préalablement purgé de traces d'eau par un courant d'argon. Un volume d'environ 100 à 500 µl du trichlorosilane approprié (H₂C=CH-(CH₂)_{N}-SiCl₃ est introduit dans le dessicateur, d'où les surfaces sont retirées après environ 12 heures (n = 6) ou 1 heure (n = 1). Au sortir les surfaces sont nettes et non-mouillantes.

Les groupes fonctionnels de ces surfaces double liaison (H₂C=CH-) peuvent être transformés en groupements carboxyles (-COOH) en trempant les lamelles traitées, comme décrit précédemment, pendant une dizaine de minutes dans une solution de 25 mg KM*n*O₄, 750 mg NaIO₄ dans 1 1 d'eau, puis en les rinçant trois fois dans de l'eau ultrapure.

Les lamelles ainsi fonctionnalisées peuvent réagir avec des protéines. Un volume de 300 µl d'une solution aqueuse (20 µg/ml) de protéines (protéine A, streptavidine, etc.) est déposé sur une lamelle fonctionnalisée en groupement (H₂C=CH-). Cette lamelle est incubée environ deux heures à température ambiante, puis rincée trois fois dans de l'eau ultrapure. Les surfaces ainsi traitées sont nettes et mouillantes. Les surfaces traitées à la protéine A peuvent ensuite réagir avec un anticorps, par exemple anti-DIG, par incubation dans une solution de 20 µg/ml d'anticorps.

Par ailleurs, sur les surfaces présentant des groupements carboxyles, on peut greffer des oligonucléotides présentant une extrémité amine (-NH₂), 200µl d'une solution de MES (50 mM, pH 55), Carbodiimide (1mg/ml) et 5µl d'oligo-aminé (10 pmole/140 µl) sont déposés sur une surface carboxylée et incubés environ 8 heures à température ambiante. La lamelle est finallement rincée trois fois dans NaOH (0.4 M) puis quatre fois dans de l'eau ultrapure. Les lamelles ainsi préparées peuvent hybrider des ADN complémentaires de l'oligonucléotide ancré.

### Ancrage d'ADN natif sur surface double liaison

Une goutte de 2 µl d'une solution d'ADN-λ marqué par fluorescence (YOYO1, POPO1 ou YOYO3, mais sans terminaison particulière) de concentration variable et dans différents tampons (nombre total de molécules < 10⁷) est déposé sur une lamelle prétraitée (C=C) et recouverte d'une lamelle de verre non traitée (diamètre 18 mm). La préparation est incubée environ 1 heure à température ambiante dans une atmosphère saturée en vapeur d'eau. Dans un tampon de 0,05 M MES (pH = 5,5), on observe un ancrage quasi-général des molécules d'ADN. Par contre dans un tampon de 0,01 M Tris (pH = 8) il n'y a presqu'aucune molécule d'ancrée (rapport > 10⁶). Cette dépendance peut permettre le contrôle de l'activation/désactivation des surfaces (vis-à-vis de l'ADN) par l'intermédiaire du pH.

L'action du ménisque sur la molécule est limitée au voisinage immédiat de celui-ci. La partie de la molécule en solution devant le ménisque fluctue librement et la partie laissée collée à la surface derrière le ménisque est insensible à un changement de direction du ménisque. Le taux d'extension de la molécule est donc uniforme et indépendant de sa taille.

### Alignement et détection de l'ancrage par l'action du ménisque

En transférant la préparation précédente dans -une atmosphère sèche, la solution en s'évaporant va étirer les molécules d'ADN, ancrées à la surface, perpendiculairement au ménisque. La force capillaire sur la molécule d'ADN (quelques dizaines de picoNewtons) est en effet suffisante pour étirer complètement la molécule (plus grande que les forces d'élasticité entropique), mais trop faible pour briser la liaison entre l'extrémité de la molécule et la surface traitée. L'ADN étant marqué par fluorescence, on observe individuellement et aisément les molécules étirées (longueur totale environ 22 µm). L'ancrage entre la surface et l'ADN étant limité aux extrémités, on a pu aussi bien étirer des ADN de phage λ, de YAC ou de E coli (longueur totale supérieure à 400 µm). Cette préparation d'ADN étirés, fluorescents et à l'air libre est stable pendant plusieurs jours et peut être observée de façon non destructive, par épifluorescence (microscope inversé Nikkon Diaphot avec objectif x100, O.N.: 1.25).

### Ancrage et détection spécifiques

En traitant les surfaces comme décrit précédemment avec un anticorps monoclonal spécifique, on peut contrôler très précisément leur spécificité. Ainsi, on a testé la spécificité de surfaces traitées anti-DIG vis-à-vis d'ADN-λ hybridés avec un oligonucléotide complémentaire d'une des extrémités Cos et possédant un groupement digoxigénine (DIG) et vis-à-vis d'ADN non hybridés. Dans le premier cas, on a observé une extension, par action du ménisque, quasi-générale des molécules ancrées. Dans le second cas, on n'a observé que quelques molécules d'ADN (< 10) ancrées dans tout l'échantillon. On estime donc que la spécificité de la méthode selon l'invention est meilleure que 10⁶.

Des ADN-λ ont aussi été hybridés avec des oligonucléotides complémentaires d'une des extrémités COS et fixés sur des surfaces carboxylées, comme décrit ci-dessus. Les conditions d'hybridation (eau pure à 40°C) n'étaient pas stringentes, car dans des conditions stringentes (haute salinité) la fluorescence des sondes YOYO1 disparaît et les ADN hybridés ne peuvent être vus. Les ADN ainsi hybridés ont aussi pu être alignés par passage du ménisque.

### Sensibilité de la détection

Afin de déterminer la sensibilité de la méthode de détection par extension du ménisque, on a déposé sur des surfaces double liaison des gouttes de 2,5 µl d'une solution d'ADN-λ dans 0,05 M MES (pH = 5,5) contenant un total de 10⁵, 10⁴ et 1 000 molécules. L'ancrage et l'alignement s'effectuent comme décrit précédemment. Les lamelles sont ensuite observées en microscopie par épifluorescence, pour déterminer la densité de molécules peignées. Celle-ci correspond bien à celle estimée : environ 4-6 molécules d'ADN par champ de vision (100 µm x 100 µm) pour un total de 10⁵ molécules d'ADN. Pour la plus faible concentration, on a pu observer une dizaine de molécules étendues par action du ménisque. Ce nombre est essentiellement limité par le grand nombre de champs de vision nécessaires à couvrir tout l'échantillon (environ 25 000), ce qui rend une recherche manuelle difficile, mais peut être avantageusement effectuée automatiquement ou aussi avec un objectif plus faible, mais à plus grand champ. En conclusion, la sensibilité de la méthode selon l'invention permet une détection et un comptage individuel de moins de 1 000 molécules d'ADN.

### Dépendance de l'étirement sur le traitement de surface

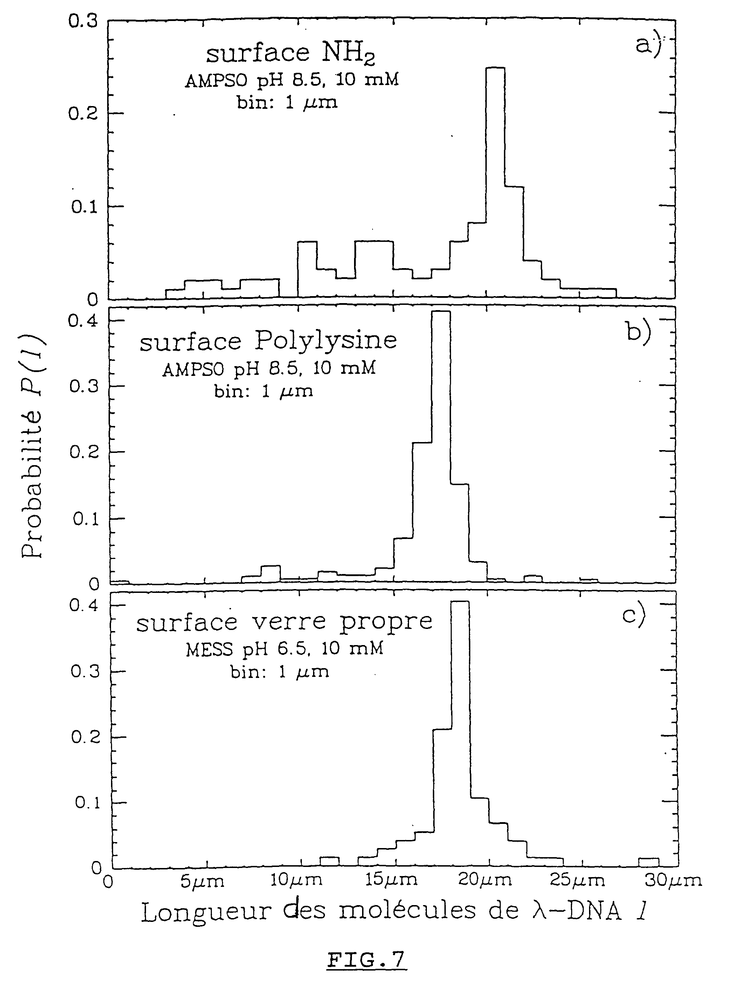

L'histogramme des longueurs d'ADN-λ, greffés sur des surfaces différentes, puis alignés par passage du ménisque montre un pic bien défini, mais différent pour les différentes surfaces. Ainsi sur des surfaces recouvertes d'un silane se terminant par un groupement vinyl l'ADN est étiré jusqu'à environ 22 µm (voir ci-dessus) pour des surfaces silanisées avec un groupement amine (-NH₂), l'histogramme présente un pic à 21 µm (Fig. 7 (a)) et sur du verre propre à environ 18.5 µm (Fig. 7(c)).

L'étirement dépend donc du traitement de surface.

### EXEMPLE 2

### Peignage de molécules d'ADN sur différentes surfaces

On a observé le peignage moléculaire de l'ADN sur des surfaces de verre traitées de différentes façons. On joue sur la différence d'adsorption entre les extrémités de la molécule et le reste de celle-ci. En adsorbant des polymères chargés positivement sur une surface de verre on favorise une adsorption des molécules d'ADN chargées négativement, cependant lorsque cette charge est grande la molécule d'ADN est collée sur toute sa longueur et le peignage est impossible. Mais il est possible de modifier la charge des polymères adsorbés sur le verre en modifiant les conditions de pH, en effet, les charges positives sont portées par exemple par des groupes *NH*_{*2*} qui passent à l'état protonné *NH*^{*+*}_{*3*} pour un pH inférieur au pK de la base correspondante. En pH basique les charges disparaissent et la surface n'attire plus l'ADN. En contrôlant finement le pH on a observé que les molécules d'ADN en solution passaient d'un état où elles sont complètement collées à la surface à une phase intermédiaire où elles ne sont ancrées que par leurs extrémités puis à une phase où la surface ne présente plus d'affinité pour l'ADN. Dans la phase intermédiaire le peignage moléculaire est réalisable.

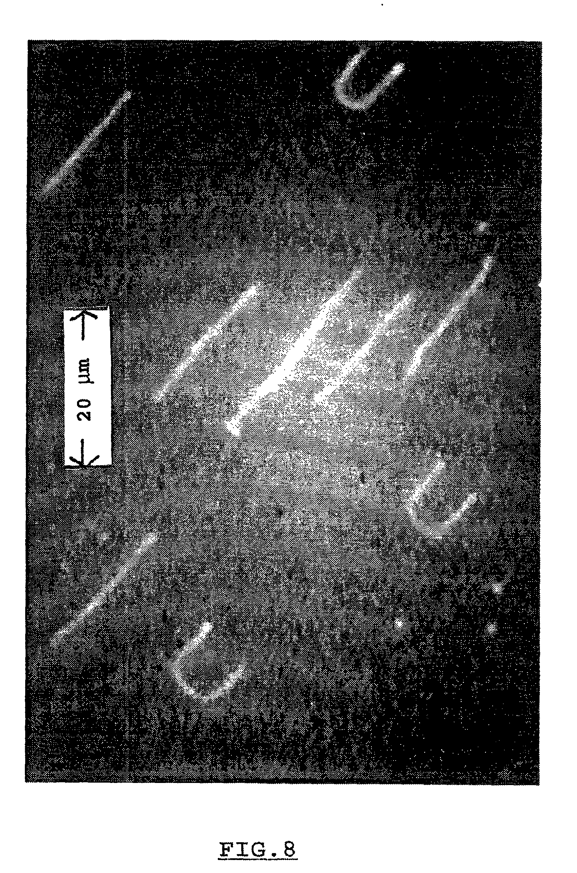

On a étudié des surfaces recouvertes d'un silàne terminé par un groupement *NH*_{*2*} pour lesquelles on observe un collage total à pH < 8, un peignage pour 8.5 < *pH* < 9.5. Le nombre de molécules peignées est maximum à pH = 8.5 il est divisé par 2 à pH = 9 et par 4 à pH = 9.5. On a aussi déterminé l'extension relative sur cette surface qui correspond à 1.26 comme on peut le voir sur l'histogramme 2 de la figure 7 qui représente des histogrammes de la longueur des molécules d'ADN λ peignées sur des surfaces de verre :
a) recouvertes de silane terminés par un groupement aminé, b)recouvertes de polylysine,
c) nettoyées dans un mélange eau oxygénée/acide sulfurique.
   On a aussi regardé des surfaces recouvertes de polylysine qui présentent des caractéristiques d'accrochages similaires en pH : domaine de peignage 8.5 et présentent une extension relative plus faible : 1.08. On peut avoir un exemple typique sur la figure 8 qui représente des molécules d'ADN peignées sur des surfaces de verre recouvertes de polylysine. On remarque que les molécules attachées par leurs deux extrémités forment des boucles.

Finalement, on a retrouvé le même comportement sur des surfaces de verre fraîchement nettoyées dans un mélange eau oxygénée / acide sulfurique concentré. Ces surfaces sont très mouillantes et se polluent rapidement, cependant, on a observé un domaine de peignage entre 5.5 < pH < 7.4 tandis que le domaine d'adsorption forte se situe à *pH = 4.5.* L'extension relative des molécules correspond à 1.12.

### EXEMPLE 3

### Alignement uniforme et directionel de YAC

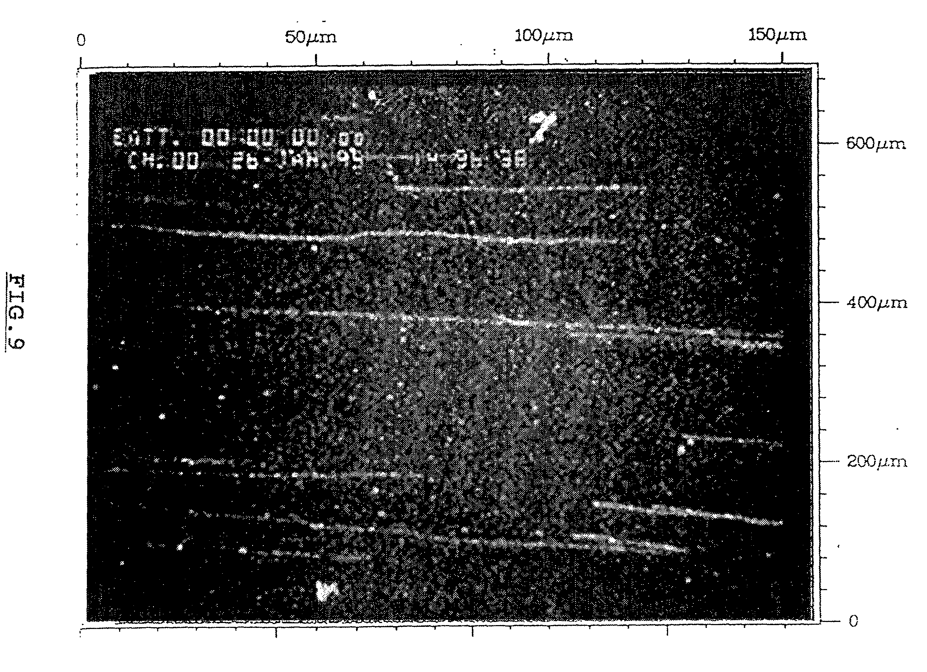

1µg de YAC préalablement teint dans son bloc d'agarose à l'aide d'une sonde fluorescente YOYO1 est chauffé à 68°C, agarasé, puis dilué dans 10 ml de MES (50mM pH 5.5). Deux lamelles silanisées (surfaces C=C) sont incubées pendant =1,5h dans cette solution puis retirées à environ 170 µm/sec. Les molécules de YAC sont toutes alignées parallèlement à la direction du retrait des lamelles (Fig. 9). L'intégrité des molécules ainsi alignées est meilleure que par évaporation après déposition entre deux lamelles.

### Hybridation d'un cosmide sur un YAC peigné

Un YAC teinté comme décrit précédemment est ancré sur une surface C=C (entre deux lamelles) puis aligné par le ménisque, lors de l'évaporation de la solution. Les sondes (cosmides) sont marquées par incorporation d'un nucléotide biotynilé par la technique de "randon priming". Les sondes marquées (100 ng) et 5 µg d'ADN de sperme de saumon soniqué (=500 bps) sont purifiées par précipitation dans Na-acétate et éthanol, puis dénaturées dans du formamide.

Les YAC peignés sont dénaturés entre deux lamelles avec 120 µl de solution dénaturante (formamide 70%, 2 x SSC) sur une plaque chauffante à 80°C pendant 3 minutes. Les sondes (20 ng) préalablement dénaturées sont déposées sur la lamelle dans une solution d'hybridation (formamide 55 %, 2 x SSC, 10 % dextran sulfate) recouvertes d'une lamelle qui est scellée avec du caoutchouc liquide (rubber cement). L'hybridation est réalisée une nuit à 37°C en chambre humide.

La révélation des hybrides se fait suivant les protocoles connus pour les hybridations in situ sur chromosomes décondensés (D. Pinkel et al., PNAS USA 83, 2934 (1986) et PNAS USA 85, 9138 (1988)).

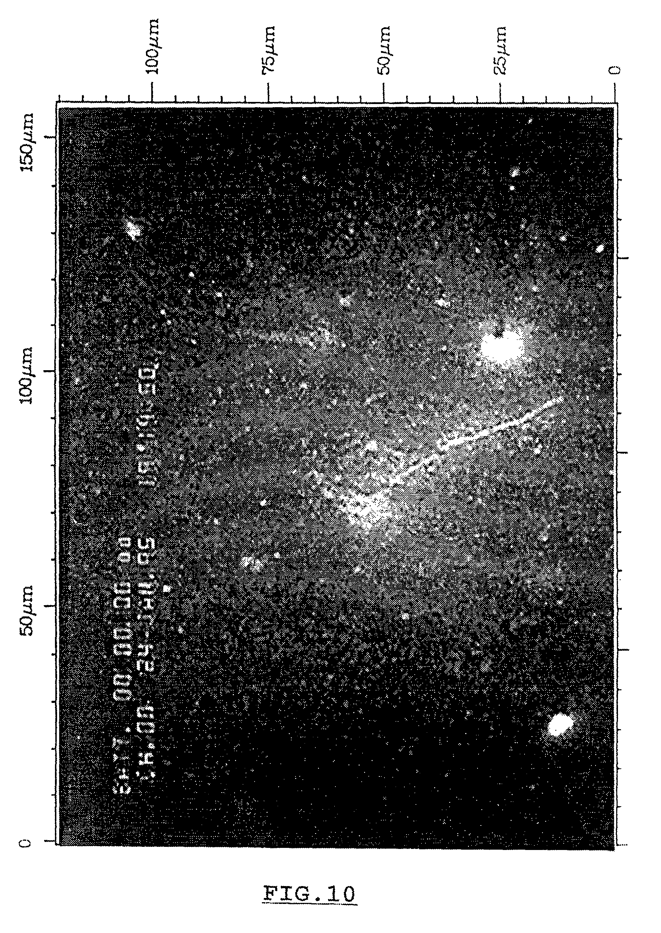

En microscopie, en fluorescence on observe alors des segments hybridés tels celui montré dans la Fig. 10. Cet exemple démontre la possibilité de détecter la présence d'un gène sur une molécule d'ADN, qui peut être utilisé à des fins de diagnostic ou de cartographie physique du génome.

## Revendications

1. Procédé d'alignement de macromolécule(s) sur la surface S d'un support, **caractérisé en ce que** l'on fait se déplacer sur ladite surface S la ligne triple S/A/B/ (ménisque) résultant du contact d'un solvant A avec la surface S et un milieu fluide ou gazeux B, lesdites macromolécules ayant une partie, notamment une extrémité, ancrée sur la surface S, l'autre partie, notamment l'autre extrémité, étant en solution dans le solvant A.

2. Procédé selon la revendication 1, **caractérisé en ce que** le déplacement du ménisque se fait par évaporation du solvant A.

3. Procédé selon la revendication 1, **caractérisé en ce que** le déplacement du ménisque se fait par déplacement relatif de l'interface A/B par rapport à la surface S.

4. Procédé selon la revendication 3, **caractérisé en ce que** pour déplacer le ménisque, la surface S est retirée du solvant A ou le solvant A est retiré de la surface S.

5. Procédé selon l'une des revendications 1 à 4, **caractérisé en ce que** le ménisque est un ménisque eau-air.

6. Procédé selon l'une des revendications 1 à 5 **caractérisé en ce que** le support est constitué au moins en surface par un polymère organique ou inorganique, un métal, un oxyde ou un sulfure de métal, un élément semi-conducteur tel que le silicium ou un oxyde d'élément semi-conducteur, ou une de leurs combinaisons.

7. Procédé selon l'une des revendications 1 à 6, **caractérisé en ce que** le support est constitué au moins en surface par du verre, du silicium oxydé en surface, de l'or, du graphite, du sulfure de molybdène ou du mica.

8. Procédé selon l'une des revendications 1 à 7, **caractérisé en ce que** le support est sous forme de plaque, de bille, de fibre ou de particules.

9. Procédé selon l'une des revendications 1 à 8, **caractérisé en ce que** le solvant A dans lequel les macromolécules à aligner sont en solution est placé entre deux supports, dont un au moins correspond audit support de surface S, et le ménisque est déplacé par évaporation.

10. Procédé selon l'une des revendications 1 à 9 **caractérisé en ce que** l'ancrage de la macromolécule se fait par interaction physicochimique, notamment adsorption ou par lien covalent, soit directement entre la surface et la macromolécule, soit indirectement entre la surface et une autre molécule reconnaissant et/ou interagissant avec ladite macromolécule.

11. Procédé selon l'une des revendications 1 à 10, **caractérisé en ce qu'**on utilise un support présentant en surface un groupement réactif exposé ayant une affinité pour ladite macromolécule ou une molécule à activité biologique capable de reconnaître ladite macromolécule.

12. Procédé selon l'une des revendications 1 à 11, **caractérisé en ce que** la surface est recouverte d'un groupe choisi parmi les groupes vinyle, amine, carboxyle, aldéhyde ou hydroxyle.

13. Procédé selon l'une des revendications 10 ou 12, **caractérisé en ce que** la surface comporte
- sur un support une couche sensiblement monomoléculaire d'un composé organique de structure allongée ayant au moins :
- un groupement de fixation présentant une affinité pour le support, et
- un groupement exposé n'ayant pas ou peu d'affinité pour ledit support et ledit groupement de fixation dans les conditions de fixation, mais présentant éventuellement, après une modification chimique suivant la fixation, une affinité pour ladite macromolécule ou molécule à activité biologique.

14. Procédé selon l'une des revendications 1 à 13 **caractérisé en ce que** l'on réalise l'ancrage d'une partie d'une macromolécule par adsorption sur une surface, en mettant ladite macromolécule en présence de ladite surface dans une zone de pH ou de teneur ionique du milieu déterminée ou en appliquant une tension électrique déterminée sur la surface d'ancrage.

15. Procédé selon les revendications 1 à 14 **caractérisé en ce que** le pH pour réaliser l'ancrage est choisi dans une plage comprise entre un pH favorisant un état d'adsorption complet et un pH favorisant une absence d'adsorption.

16. Procédé selon l'une des revendications 1 à 15 dans lequel on réalise l'ancrage d'un acide nucléique ou d'une protéine par adsorption sur une surface présentant des groupes comportant des doubles liaisons éthyléniques ou des groupes amines, en mettant l'acide nucléique ou la protéine en présence de la surface dans une zone de pH ou de teneur ionique du milieu, déterminée.

17. Procédé selon la revendication 16 **caractérisé en ce qu'**on réalise l'ancrage d'ADN non fonctionnalisée par adsorption sur des surfaces recouvertes de molécules terminées par un groupement vinyl ou amine.

18. Procédé selon la revendication 17 dans lequel on réalise l'ancrage de l'ADN par son extrémité sur une surface présentant des groupements à double liaison éthylénique, en mettant l'ADN en présence de la surface à un pH inférieur à 8.

19. Procédé selon la revendication 18, **caractérisé en ce que** la réaction est conduite à un pH compris entre 5 et 6 puis est stoppée à pH 8.

20. Procédé selon la revendication 17, **caractérisé en ce qu'**on réalise l'ancrage d'ADN par son extrémité sur une surface recouverte de polylysine ou d'un groupement silane terminé par un groupe amine.

21. Procédé selon la revendication 17, **caractérisé en ce qu'**on réalise l'ancrage de l'ADN par son extrémité sur une surface recouverte par un groupement amine en mettant l'ADN en présence de la surface à pH entre 8 et 10.

22. Procédé selon la revendication 17, **caractérisé en ce qu'**on réalise l'ancrage d'ADN par son extrémité sur une surface de verre traité auparavant dans un bain d'acide, en mettant l'ADN en présence de ladite surface à pH entre 5 et 8.

23. Procédé de mise en évidence, de séparation et/ou de dosage d'une macromolécule dans un échantillon, **caractérisé en ce qu'**on utilise un procédé d'alignement selon l'une des revendications 1 à 22 dans lequel se trouve fixée sur la surface S une molécule à activité biologique capable de reconnaître ladite macromolécule de l'échantillon et **en ce que** la mise en évidence, la séparation ou le dosage sont effectués grâce à un réactif fluorescent ou non détectant la présence de la molécule fixée ou ladite macromolécule.

24. Procédé selon l'une des revendications 1 à 23, **caractérisé en ce que** lesdites macromolécule et molécule à activité biologique sont choisies parmi les protéines, les acides nucléiques, les lipides, les polysaccharides et leurs dérivés.

25. Procédé selon la revendication 23, **caractérisé en ce que** lesdites macromolécule et molécule à activité biologique sont choisies parmi, les anticorps, les antigènes, les ADN et ARN, les ligands ou leurs récepteurs ainsi que leurs dérivés.

26. Procédé selon les revendications 23 à 25, **caractérisé en ce que** l'ADN fixé comporte la séquence complémentaire d'une séquence d'ADN à isoler d'un échantillon.

27. Procédé selon les revendications 23 à 25, **caractérisé en ce que** la protéine fixée est capable de reconnaître et fixer spécifiquement une protéine à isoler d'un échantillon.

28. Procédé selon les revendications 23 à 25, **caractérisé en ce que** ladite molécule à activité biologique est choisie parmi la biotine, l'avidine, la streptavidine, leurs dérivés ou un système antigène-anticorps.

29. Procédé selon les revendications 23 à 25, **caractérisée en ce que** la surface est à faible fluorescence et **en ce que** le réactif est fluorescent.

30. Procédé selon les revendications 23 à 25, **caractérisée en ce que** le réactif est constitué par des billes.

31. Procédé selon l'une des revendications 23 à 25, **caractérisé en ce que** la détection est faite par microscopie optique ou à champs proche.

32. Procédé selon l'une des revendications 23 à 25, **caractérisé en ce qu'**on soumet le produit de réaction entre la molécule à activité biologique et la macromolécule de l'échantillon à une contrainte afin de détruire les mauvais appariements avant la détection.

33. Procédé de mise en évidence d'une macromolécule consistant en une séquence d'ADN ou d'une protéine dans un échantillon, selon l'une des revendications 23 à 32, **caractérisé en ce que** :
- on met l'échantillon correspondant au solvant A, dans lequel ladite macromolécule est en solution, en contact avec la surface du support dans des conditions de formation d'un hybride ADN/ADN, ADN/ARN ou de formation du produit de réaction protéine/protéine,
- l'hybride ou une macromolécule de marquage de l'hybride ou du produit de réaction étant ancré en une partie, le reste étant en solution, on l'étire par déplacement du ménisque créé par le contact du solvant avec la surface pour orienter les hybrides ou lesdites macromolécules de marquage et on effectue la mesure ou l'observation des hybrides ou desdites macromolécules de marquage ainsi orientés.

34. Procédé selon l'une des revendications 23 à 33, **caractérisé en ce que** l'ADN fixé et l'ADN de l'échantillon sont « colorés » de façon différente et, après étirement, on mesure la position de la séquence complémentaire par rapport à l'extrémité de l'ADN de l'échantillon.

35. Procédé selon l'une des revendications 23 à 34, **caractérisé en ce qu'**on utilise une méthode de détection ELISA ou FISH.

36. Procédé selon l'une des revendications 23 à 35, **caractérisé en ce que** l'échantillon est le produit ou le substrat d'une amplification enzymatique d'acide nucléique.

37. Procédé selon l'une des revendications 23 à 36, **caractérisé en ce que** l'ADN est étiré, puis dénaturé puis hybridé avec des sondes spécifiques pour déterminer la position ou la taille d'une ou plusieurs séquences d'ADN déterminées.

38. Procédé de cartographie physique d'un gène sur un ADN génomique dans lequel l'ADN est aligné et/ou mis en évidence selon un procédé d'une des revendications 1 à 37.

39. Procédé selon la revendication 38, **caractérisé en ce que** la position et la taille du gène recherché sur l'ADN génomique sont déterminées par l'hybridation avec des sondes spécifiques dudit gène à cartographier.

40. Procédé selon l'une des revendications 33 à 36, **caractérisé en ce que** l'ADN est étiré, puis dénaturé puis hybridé avec des sondes spécifiques pour déterminer la présence ou l'absence d'une ou plusieurs séquences d'ADN données.

41. Procédé de diagnostic d'une pathologie lié à la présence ou à l'absence d'une séquence d'ADN donnée spécifique de ladite pathologie dans lequel on utilise un procédé selon la revendication 40.

42. Procédé de préparation d'un gène à partir d'ADN génomique **caractérisé en ce que** l'on identifie la position dudit gène sur l'ADN génomique aligné par le procédé de l'une des revendications 1 à 36 à l'aide d'une sonde spécifique dudit gène et on procède à l'amplification enzymatique de la séquence dudit gène et/ou de ses séquences flanquantes et on isole le produit amplifié.

43. Utilisation d'un gène obtenu par un procédé selon la revendication 42 pour préparer une construction d'ADN utile dans un procédé de remplacement d'un gène dans le génome d'une cellule eucaryote par insertion ciblée d'un gène étranger, ledit gène étranger étant obtenu par le procédé de la revendication 42.

## Patentansprüche

1. Verfahren zur Ausrichtung von einem oder mehreren Makromolekül(en) auf der Oberfläche S eines Trägers, **dadurch gekennzeichnet, dass** man auf der Oberfläche S die Tripel-Linie S/A/B/ (Meniskus), welche aus dem Kontakt eines Lösemittels A mit der Oberfläche S und einem fluiden oder gasförmigen Medium B resultiert, sich verschieben lässt, wobei die Makromoleküle einen Abschnitt, insbesondere ein Ende, haben, der auf der Oberfläche S verankert ist, wobei der andere Abschnitt, insbesondere das andere Ende, sich in Lösung in dem Lösemittel A befindet.

2. Verfahren nach Anspruch 1, **dadurch gekennzeichnet, dass** die Verschiebung des Meniskus durch Verdampfen des Lösemittels A erfolgt.

3. Verfahren nach Anspruch 1, **dadurch gekennzeichnet, dass** die Verschiebung des Meniskus durch Verschiebung bezüglich der Grenzfläche A/B bezogen auf die Oberfläche S erfolgt.

4. Verfahren nach Anspruch 3, **dadurch gekennzeichnet, dass** für das Verschieben des Meniskus die Oberfläche S von dem Lösemittel A zurückgezogen wird oder das Lösemittel A von der Oberfläche S zurückgezogen wird.

5. Verfahren nach einem der Ansprüche 1 bis 4, **dadurch gekennzeichnet, dass** der Meniskus ein Wasser-Luft-Meniskus ist.

6. Verfahren nach einem der Ansprüche 1 bis 5, **dadurch gekennzeichnet, dass** der Träger wenigstens an der Oberfläche aus einem organischen oder anorganischen Polymer, einem Metall, einem Metalloxid oder -sulfid, einem Halbleiterelement, wie Silicium, oder einem Halbleiterelementoxid oder einer von deren Kombinationen gebildet wird.

7. Verfahren nach einem der Ansprüche 1 bis 6, **dadurch gekennzeichnet, dass** der Träger wenigstens an der Oberfläche aus Glas, aus an der Oberfläche oxidiertem Silicium, aus Gold, Graphit, Molybdänsulfid oder Glimmer gebildet wird.

8. Verfahren nach einem der Ansprüche 1 bis 7, **dadurch gekennzeichnet, dass** der Träger in Platten-, Kugel-, Faser- oder Teilchenform vorliegt.

9. Verfahren nach einem der Ansprüche 1 bis 8, **dadurch gekennzeichnet, dass** das Lösemittel A, in welchem die auszurichtenden Makromoleküle sich in Lösung befinden, zwischen zwei Trägern, von denen wenigstens einer dem Träger mit der Oberfläche S entspricht, angeordnet ist und der Meniskus durch Verdampfung verschoben wird.

10. Verfahren nach einem der Ansprüche 1 bis 9, **dadurch gekennzeichnet, dass** die Verankerung des Makromoleküls durch physikalisch-chemische Wechselwirkung, insbesondere Adsorption, oder durch kovalente Bindung entweder direkt zwischen der Oberfläche und dem Makromolekül oder indirekt zwischen der Oberfläche und einem anderen Molekül, das das Makromolekül erkennt und/oder mit diesem wechselwirkt, erfolgt.

11. Verfahren nach einem der Ansprüche 1 bis 10, **dadurch gekennzeichnet, dass** man einen Träger einsetzt, der an der Oberfläche eine exponierte reaktive Gruppe mit einer Affinität für das Makromolekül oder ein Molekül mit biologischer Aktivität, das in der Lage ist, das Makromolekül zu erkennen, aufweist.

12. Verfahren nach einem der Ansprüche 1 bis 11, **dadurch gekennzeichnet, dass** die Oberfläche mit einer Gruppe, ausgewählt unter den Vinyl-, Amin-, Carboxyl-, Aldehyd- oder Hydroxylgruppen, bedeckt ist.

13. Verfahren nach einem der Ansprüche 10 oder 12, **dadurch gekennzeichnet, dass** die Oberfläche umfasst:
- auf einem Träger eine im wesentlichen monomolekulare Schicht aus einer organischen Verbindung mit langgestreckter Struktur, welche wenigstens aufweist:
- eine Anheftungsgruppe, die eine Affinität für den Träger aufweist, und
- eine exponierte Gruppe, die keine oder nur geringe Affinität für den Träger und die Anheftungsgruppe unter den Anheftungsbedingungen aufweist, aber, gegebenenfalls nach einer chemischen Modifizierung nach der Anheftung, eine Affinität für das Makromolekül oder Molekül mit biologischer Aktivität aufweist.

14. Verfahren nach einem der Ansprüche 1 bis 13, **dadurch gekennzeichnet, dass** man die Verankerung eines Abschnitts eines Makromoleküls durch Adsorption auf eine Oberfläche ausführt, indem man das Makromolekül in einem bestimmten pH- oder Ionengehalt-Bereich des Mediums in Kontakt mit der Oberfläche bringt oder indem man eine bestimmte elektrische Spannung an die Verankerungsoberfläche anlegt.

15. Verfahren nach den Ansprüchen 1 bis 14, **dadurch gekennzeichnet, dass** der pH zur Ausführung der Verankerung in einem Bereich zwischen einem pH, der einen Zustand vollständiger Adsorption begünstigt, und einem pH, der ein Fehlen von Adsorption begünstigt, ausgewählt wird.

16. Verfahren nach einem der Ansprüche 1 bis 15, bei welchem man die Verankerung einer Nukleinsäure oder eines Proteins durch Adsorption auf eine Oberfläche, die ethylenische Doppelbindungen umfassende Gruppen oder Amingruppen aufweist, ausführt, indem man die Nukleinsäure oder das Protein in einem bestimmten pH- oder Ionengehalt-Bereich des Mediums mit der Oberfläche in Kontakt bringt.

17. Verfahren nach Anspruch 16, **dadurch gekennzeichnet, dass** man die Verankerung von nicht-funktionalisierter DNA durch Adsorption auf Oberflächen, die von Molekülen, deren Enden aus einer Vinyl- oder Amingruppe bestehen, bedeckt sind, ausführt.

18. Verfahren nach Anspruch 17, bei welchem man die Verankerung der DNA durch ihr Ende auf einer Oberfläche, die Gruppen mit ethylenischen Doppelbindungen aufweist, ausführt, indem man die DNA mit der Oberfläche bei einem pH unter 8 in Kontakt bringt.

19. Verfahren nach Anspruch 18, **dadurch gekennzeichnet, dass** die Umsetzung bei einem pH zwischen 5 und 6 ausgeführt, dann bei pH 8 gestoppt wird.

20. Verfahren nach Anspruch 17, **dadurch gekennzeichnet, dass** man die DNA-Verankerung durch ihr Ende auf einer Oberfläche, die mit Polylysin oder einer Silangruppierung, deren Ende eine Amingruppe bildet, bedeckt ist, ausführt.

21. Verfahren nach Anspruch 17, **dadurch gekennzeichnet, dass** man die Verankerung der DNA durch ihr Ende auf einer Oberfläche, die mit einer Amingruppe bedeckt ist, ausführt, indem man die DNA mit der Oberfläche bei einem pH zwischen 8 und 10 in Kontakt bringt.

22. Verfahren nach Anspruch 17, **dadurch gekennzeichnet, dass** man die DNA-Verankerung durch ihr Ende auf einer zuvor in einem Säurebad behandelten Glasoberfläche ausführt, indem man die DNA mit der Oberfläche bei einem pH zwischen 5 und 8 in Kontakt bringt.

23. Verfahren zum Nachweis, zur Abtrennung und/oder zur quantitativen Bestimmung eines Makromoleküls in einer Probe, **dadurch gekennzeichnet, dass** man ein Ausrichtungsverfahren nach einem der Ansprüche 1 bis 22 einsetzt, bei welchem sich an der Oberfläche S ein Molekül mit biologischer Aktivität, welches in der Lage ist, das Makromolekül der Probe zu erkennen, angeheftet befindet, und **dass** der Nachweis, die Abtrennung oder die quantitative Bestimmung mit Hilfe eines fluoreszierenden oder nicht-fluoreszierenden Reagens, das die Anwesenheit des angehefteten Moleküls oder des Makromoleküls detektiert, ausgeführt werden.

24. Verfahren nach einem der Ansprüche 1 bis 23, **dadurch gekennzeichnet, dass** das Makromolekül und das Molekül mit biologischer Aktivität unter den Proteinen, den Nukleinsäuren, den Lipiden, den Polysacchariden und deren Derivaten ausgewählt werden.

25. Verfahren nach Anspruch 23, **dadurch gekennzeichnet, dass** das Makromolekül und das Molekül mit biologischer Aktivität unter den Antikörpern, den Antigenen, den DNA und RNA, den Liganden oder deren Rezeptoren wie auch deren Derivaten ausgewählt werden.

26. Verfahren nach den Ansprüchen 23 bis 25, **dadurch gekennzeichnet, dass** die angeheftete DNA die zu einer Sequenz von aus einer Probe zu isolierender DNA komplementäre Sequenz umfasst.

27. Verfahren nach den Ansprüchen 23 bis 25, **dadurch gekennzeichnet, dass** das angeheftete Protein in der Lage ist, ein aus einer Probe zu isolierendes Protein spezifisch zu erkennen und zu binden.

28. Verfahren nach den Ansprüchen 23 bis 25, **dadurch gekennzeichnet, dass** das Molekül mit biologischer Aktivität unter Biotin, Avidin, Streptavidin, deren Derivaten oder einem Antigen-Antikörper-System ausgewählt wird.

29. Verfahren nach den Ansprüchen 23 bis 25, **dadurch gekennzeichnet, dass** die Oberfläche schwach fluoreszierend ist und dass das Reagens fluoreszierend ist.

30. Verfahren nach den Ansprüchen 23 bis 25, dadurch gekenn- zeichnet, dass das Reagens durch Kugeln gebildet wird.

31. Verfahren nach einem der Ansprüche 23 bis 25, **dadurch gekennzeichnet, dass** der Nachweis durch optische Mikroskopie oder bei einem Nahfeld erfolgt.

32. Verfahren nach einem der Ansprüche 23 bis 25, **dadurch gekennzeichnet, dass** man das Reaktionsprodukt zwischen dem Molekül mit biologischer Aktivität und dem Makromolekül der Probe einer Beanspruchung unterwirft, um die schlechten Paarungen vor dem Nachweis zu zerstören.

33. Verfahren zum Nachweis eines Makromoleküls, bestehend aus einer DNA-Sequenz oder einem Protein in einer Probe, gemäß einem der Ansprüche 23 bis 32, **dadurch gekennzeichnet, dass**:
- man die Probe, entsprechend dem Lösemittel A, in welchem sich das Makromolekül in Lösung befindet, in Kontakt mit der Oberfläche des Trägers bringt unter Bedingungen für die Bildung eines DNA/DNA-, DNA/RNA-Hybrids oder für die Bildung des Produkts aus der Protein/Protein-Reaktion,
- man, wobei das Hybrid oder ein Markierungs-Makromolekül des Hybrids oder des Reaktionsprodukts in einem Abschnitt verankert ist, wobei der Rest sich in Lösung befindet, dieses streckt durch Verschieben des Meniskus, der durch den Kontakt des Lösemittels mit der Oberfläche erzeugt wird, um die Hybride oder die Markierungs-Makromoleküle zu orientieren, und man die Messung oder die Beobachtung der so orientierten Hybride oder Markierungs-Makromoleküle vornimmt.

34. Verfahren nach einem der Ansprüche 23 bis 33, **dadurch gekennzeichnet, dass** die angeheftete DNA und die DNA der Probe auf unterschiedliche Weise "gefärbt" sind und man nach dem Strecken die Lage der komplementären Sequenz bezogen auf das Ende der DNA der Probe misst.

35. Verfahren nach einem der Ansprüche 23 bis 34, **dadurch gekennzeichnet, dass** man eine ELISA- oder FISH-Nachweismethode einsetzt.

36. Verfahren nach einem der Ansprüche 23 bis 35, **dadurch gekennzeichnet, dass** die Probe das Produkt oder das Substrat einer enzymatischen Nukleinsäureamplifizierung ist.

37. Verfahren nach einem der Ansprüche 23 bis 36, **dadurch gekennzeichnet, dass** die DNA gestreckt, dann denaturiert, dann mit spezifischen Sonden hybridisiert wird, um die Lage oder die Größe von einer oder mehreren bestimmten DNA-Sequenzen zu bestimmen.

38. Verfahren zur physischen Kartierung oder Restriktionskartierung eines Gens auf einer genomischen DNA, bei welchem die DNA ausgerichtet und/oder nachgewiesen wird gemäß einem Verfahren von einem der Ansprüche 1 bis 37.

39. Verfahren nach Anspruch 38, **dadurch gekennzeichnet, dass** die Lage und die Größe des untersuchten Gens auf der genomischen DNA durch die Hybridisierung mit für das zu kartierende Gen spezifischen Sonden bestimmt werden.

40. Verfahren nach einem der Ansprüche 33 bis 36, **dadurch gekennzeichnet, dass** die DNA gestreckt, dann denaturiert, dann mit spezifischen Sonden hybridisiert wird, um das Vorhandensein oder das Fehlen von einer oder mehreren gegebenen DNA-Sequenzen zu bestimmen.

41. Verfahren zur Diagnose einer Pathologie, welche mit dem Vorhandensein oder mit dem Fehlen einer gegebenen DNA-Sequenz, welche für die Pathologie spezifisch ist, verbunden ist, bei welchem man ein Verfahren nach Anspruch 40 einsetzt.

42. Verfahren zur Herstellung eines Gens ausgehend von genomischer DNA, **dadurch gekennzeichnet, dass** man die Lage dieses Gens auf der durch das Verfahren von einem der Ansprüche 1 bis 36 ausgerichteten genomischen DNA mit Hilfe einer für das Gen spezifischen Sonde identifiziert und man die enzymatische Amplifizierung der Sequenz dieses Gens und/oder von dessen flankierenden Sequenzen vornimmt und man das amplifizierte Produkt isoliert.

43. Verwendung eines Gens, das durch ein Verfahren nach Anspruch 42 erhalten worden ist, zur Herstellung eines DNA-Konstrukts, das in einem Verfahren zum Ersetzen eines Gens in dem Genom einer eukaryotischen Zelle durch gezielte Insertion eines Fremdgens nützlich ist, wobei das Fremdgen durch das Verfahren des Anspruchs 42 erhalten wird.

## Claims

1. Process for aligning a macromolecule (macromolecules) on the surface S of a support, **characterized in that** the triple line S/A/B (meniscus) resulting from the contact between a solvent A and the surface S and a fluid or gaseous medium B is caused to move on the said surface S, the said macromolecules having a part, especially an end, anchored on the surface S, the other part, especially the other end, being in solution in the solvent A.

2. Process according to Claim 1, **characterized in that** the movement of the meniscus is achieved by evaporation of the solvent A.

3. Process according to Claim 1, **characterized in that** the movement of the meniscus is achieved by relative movement of the A/B interface relative to the surface S.

4. Process according to Claim 3, **characterized in that** in order to move the meniscus, the surface S is removed from the solvent A or the solvent A is removed from the surface S.

5. Process according to one of Claims 1 to 4, **characterized in that** the meniscus is a water/air meniscus.

6. Process according to one of Claims 1 to 5, **characterized in that** the support consists, at least at the surface, of an organic or inorganic polymer, a metal, a metal oxide or sulphide, a semiconductor element such as silicon or an oxide of a semiconductor element, or one of the combinations thereof.

7. Process according to one of Claims 1 to 6, **characterized in that** the support consists, at least at the surface, of glass, surface-oxidized silicon, gold, graphite, molybdenum sulphide or mica.

8. Process according to one of Claims 1 to 7, **characterized in that** the support is in the form of a plate, bead, fibre or particle system.

9. Process according to one of Claims 1 to 8, **characterized in that** the solvent A in which the macromolecules to be aligned are in solution is placed between two supports, of which one at least corresponds to the said support of surface S, and the meniscus is moved by evaporation.

10. Process according to one of Claims 1 to 9, **characterized in that** the anchoring of the macromolecule is carried out by physicochemical interaction, especially by adsorption or by covalent linkage, either directly between the surface and the macromolecule, or indirectly between the surface and another molecule recognizing and/or interacting with the said macromolecule.

11. Process according to one of Claims 1 to 10, **characterized in that** a support is used which has at the surface an exposed reactive group having an affinity for the said macromolecule or a molecule with biological activity capable of recognizing the said macromolecule.

12. Process according to one of Claims 1 to 11, **characterized in that** the surface is coated with a group chosen from the vinyl, amine, carboxyl, aldehyde or hydroxyl groups.

13. Process according to either of Claims 10 and 12, **characterized in that** the surface contains:
- on a support, a substantially monomolecular layer of an organic compound of elongated structure having at least:
• an attachment group having an affinity for the support, and
• an exposed group having no or little affinity for the said support and the said attachment group under attachment conditions, but optionally having, after chemical modification following the attachment, an affinity for the said macromolecule or molecule with biological activity.

14. Process according to one of Claims 1 to 13, **characterized in that** the anchoring of a part of a macromolecule is carried out by adsorption on a surface, by bringing the said macromolecule into contact with the said surface in a determined region of pH or ionic content of the medium or by applying a determined electric voltage on the anchoring surface.

15. Process according to Claims 1 to 14, **characterized in that** the pH for carrying out the anchoring is chosen in a range between a pH favouring a state of complete adsorption and a pH favouring absence of adsorption.

16. Process according to one of Claims 1 to 15, in which the anchoring of a nucleic acid or of a protein is carried out by adsorption on a surface having groups containing ethylenic double bonds or amine groups, by bringing the nucleic acid or the protein into contact with the surface in a determined region of pH or ionic content of the medium.

17. Process according to Claim 16, **characterized in that** the anchoring of nonfunctionalized DNA is carried out by adsorption on surfaces coated with molecules ending with a vinyl or amine group.

18. Process according to Claim 17, in which the anchoring of the DNA is carried out by its end onto a surface having groups with ethylenic double bonds, by bringing the DNA into contact with the surface at a pH of less than 8.

19. Process according to Claim 18, **characterized in that** the reaction is carried out at a pH of between 5 and 6, and is then stopped at pH 8.

20. Process according to Claim 17, **characterized in that** the anchoring of DNA is carried out by its end onto a surface coated with polylysine or a silane group "ending with an amine group.

21. Process according to Claim 17, **characterized in that** the anchoring of the DNA is carried out by its end onto a surface coated with an amine group by bringing the DNA into contact with the surface at a pH of between 8 and 10.

22. Process according to Claim 17, **characterized in that** the anchoring of DNA is carried out by its end onto a glass surface treated beforehand in an acid bath, by bringing the DNA into contact with the said surface at a pH of between 5 and 8.

23. Process for detecting, separating and/or assaying a macromolecule in a sample, **characterized in that** a process for alignment according to one of Claims 1 to 22 is used in which a molecule with biological activity capable of recognizing the said sample macromolecule becomes attached to the surface S, and **in that** the detection, separation or assay are carried out using a reagent, fluorescent or otherwise, which detects the presence of the attached molecule or the said macromolecule.

24. Process according to one of Claims 1 to 23, **characterized in that** the said macromolecule and molecule with biological activity are chosen from proteins, nucleic acids, lipids, polysaccharides and derivatives thereof.

25. Process according to Claim 23, **characterized in that** the said macromolecule and molecule with biological activity are chosen from antibodies, antigens, DNAs, RNAs, ligands or their receptors as well as derivatives thereof.

26. Process according to Claims 23 to 25, **characterized in that** the attached DNA contains the sequence complementary to a DNA sequence to be isolated from a sample.

27. Process according to Claims 23 to 25, **characterized in that** the attached protein is capable of specifically recognizing and attaching a protein to be isolated from a sample.

28. Process according to Claims 23 to 25, **characterized in that** the said molecule with biological activity is chosen from biotin, avidin, streptavidin, derivatives thereof or an antigen-antibody system.

29. Process according to Claims 23 to 25, **characterized in that** the surface exhibits low fluorescence and **in that** the reagent is fluorescent.

30. Process according to Claims 23 to 25, **characterized in that** the reagent consists of beads.

31. Process according to one of Claims 23 to 25, **characterized in that** the detection is performed by optical or near field microscopy.

32. Process according to one of Claims 23 to 25, **characterized in that** the product of a reaction between the molecule with biological activity and the macromolecule of the sample is subjected to stress in order to destroy the mismatches before the detection.

33. Process for detecting a macromolecule consisting of a DNA sequence or a protein in a sample, according to one of Claims 23 to 32, **characterized in that**:
- the sample corresponding to solvent A, in which the said macromolecule is in solution, is brought into contact with the surface of the support under conditions for forming a DNA/DNA, DNA/RNA hybrid or for forming the protein/protein reaction product,
- the hybrid or a macromolecule for labelling the hybrid or the reaction product being anchored in one part, the remainder being in solution, it is stretched by the movement of the meniscus created by the contact between the solvent
and the surface in order to orient the hybrids or the said labelling macromolecules and the measurement or the observation of the hybrids or of the said labelling macromolecules thus orientated is carried out.

34. Process according to one of Claims 23 to 33, **characterized in that** the attached DNA and the sample DNA are "coloured" differently and, after stretching, the position of the complementary sequence relative to the end of the sample DNA is measured.

35. Process according to one of Claims 23 to 34, **characterized in that** an ELISA or FISH detection method is used.

36. Process according to one of Claims 23 to 35, **characterized in that** the sample is the product or the substrate of an enzymatic amplification of nucleic acid.

37. Process according to one of Claims 23 to 36, **characterized in that** the DNA is stretched, then denatured and hybridized with specific probes in order to determine the position or the size of one or more determined DNA sequences.

38. Process for the physical mapping of a gene on a genomic DNA in which the DNA is aligned and/or detected according to a process of one of Claims 1 to 37.

39. Process according to Claim 38, **characterized in that** the position and the size of the desired gene on the genomic DNA are determined by hybridization with probes specific for the said gene to be mapped.

40. Process according to one of Claims 33 to 36, **characterized in that** the DNA is stretched, then denatured and hybridized with specific probes in order to determine the presence or the absence of one or more given DNA sequences.

41. Process for diagnosing a pathology related to the presence or to the absence of a given DNA sequence specific for the said pathology, in which a process according to Claim 40 is used.

42. Process for preparing a gene from a genomic DNA, **characterized in that** the position of the said gene on the genomic DNA aligned by the process of one of Claims 1 to 36 is identified by means of a probe specific for the said gene and the enzymatic amplification of the sequence of the said gene and/or of its flanking sequences is carried out and the amplified product is isolated.

43. Use of a gene obtained by a process according to Claim 42, for preparing a DNA construct useful in a process for replacing a gene in the genome of an eukaryotic cell by targeted insertion of a foreign gene, the "said "foreign gene being obtained by the process of Claim 42.
